# EUROPEAN PATENT APPLICATION

(11) **EP 3 758 010 A1**
(43) Date of publication of application: **30.12.2020**
(21) Application number: 20181922.4
(22) Date of filing: 24.06.2020
(51) Int. Cl.: G16H 20/30, G16H 40/20

(54) **TRAINING ASSISTANTS RETRIEVAL APPARATUS, SYSTEM, METHOD, AND PROGRAM**

(30) Priority: 28.06.2019 JP 2019121549
(71) Applicant: TOYOTA JIDOSHA KABUSHIKI KAISHA, TOYOTA-SHI, AICHI-KEN 471-8571 (JP)
(72) Inventor: OTSUKI, Nobuhisa, Aichi-ken, 471-8571 (JP); NAKASHIMA, Issei, Aichi-ken, 471-8571 (JP); YAMAMOTO, Manabu, Aichi-ken, 471-8571 (JP)
(74) Representative: D Young & Co LLP

(57) **Abstract**

To retrieve a rehabilitation training assistant or a rehabilitation facility to which the training assistant belongs in order for a trainee to effectively undergo rehabilitation, a retrieval apparatus (50) includes a receiving unit (521) that receives needs regarding training to be performed by a trainee to restore or maintain his/her physical ability, an acquisition unit (522) that acquires track record information (511) of a plurality of training assistants with regard to assistance in training from an outside, a retrieval unit (524) that retrieves a plurality of potential training assistants who meet the needs based on the track record information, and an output unit (526) that outputs information based on the plurality of potential training assistants.

## Description

### BACKGROUND

The present disclosure relates to a retrieval apparatus, system, method, and program.

Some patients use a rehabilitation support device such as a gait training device when undergoing rehabilitation (rehab, training) in order to restore or maintain his/her physical ability. As an example of the gait training device, Japanese Unexamined Patent Application Publication No. 2015-223294 discloses a gait training device that includes a walking assist device which is to be worn on the leg of a trainee and assists them in walking.

In rehabilitation using the rehabilitation support device, a training staff (trainer, training assistant), such as a doctor or a physical therapist, is there to assist a trainee, and they talk to and help the trainee, and sometimes make settings of the rehabilitation support device.

### SUMMARY

While patients suffering from a serious illness or a severe injury undergo rehabilitation in a medical institution where they had surgery during an acute phase immediately after surgery, they generally continue rehabilitation in another rehabilitation facility during a recovery phase. Further, elderly people, etc. undergo rehabilitation in a rehabilitation facility in order to try to maintain the physical ability. However, due to a difference in the area of specialty in rehabilitation and a difference in the skill of physical therapists and so on, it is difficult to find a training assistant who meets the needs of a trainee in rehabilitation or a rehabilitation facility to which this training assistant belongs.

The present disclosure has been accomplished to solve the above problems and an object of the present invention is thus to provide a retrieval apparatus, system, method, and program for retrieving a rehabilitation training assistant or a rehabilitation facility to which the training assistant belongs in order for a trainee to effectively undergo rehabilitation.

A retrieval apparatus according to a first aspect of the present disclosure includes a receiving unit configured to receive needs regarding training to be performed by a trainee to restore or maintain his/her physical ability, a retrieval unit configured to retrieve a plurality of potential training assistants who meet the needs based on track record information of a plurality of training assistants with regard to assistance in the training, and an output unit configured to output information based on the plurality of potential training assistants.

As described above, in the first aspect, by identifying potential training assistants who more suit the needs in training by taking a difference in the area of specialty of each training assistant or a difference in the skill on a body part rehabilitated into consideration based on the track record information of training assistants such as physical therapists with regard to assistance in the training, it is possible to identify training assistants who are likely to achieve a training target of a trainee or rehabilitation facilities to which each of the training assistants belongs. It is thereby possible to retrieve rehabilitation training assistants or rehabilitation facilities to which the training assistants belong in order for a trainee to effectively undergo rehabilitation.

Further, the retrieval apparatus may further include a storage unit configured to store facility information for performing the training, indicating a facility to which each of the plurality of training assistants belongs, the retrieval unit may identify a facility to which each of the plurality of potential training assistants belongs by referring to the facility information, and the output unit may output a list of facilities indicated by the identified facility. A person on the trainee side can thereby more clearly know rehabilitation facilities to which each of training assistants who are likely to meet the needs in training belongs.

Further, the retrieval apparatus may further include an evaluation value calculation unit configured to calculate an evaluation value for each facility by performing predetermined statistical processing based on the track record information of each of the potential training assistants, and the output unit may output the list of the facilities in accordance with an order of the evaluation values. It is thereby possible to calculate the evaluation value for each facility in accordance with the track record information of each of the potential training assistants who are likely to meet the needs in training and thereby grasp the degree of meeting the needs in training for each facility.

Further, the retrieval apparatus may further include an index value calculation unit configured to calculate, for each of the training assistants, an index value of assisting ability related to the needs from the track record information, and the evaluation value calculation unit may calculate the evaluation value for each facility by performing the statistical processing of the index value of each of the potential training assistants. Because the assisting ability of one training assistant is different for different needs, it is possible to evaluate facilities more appropriately by using the index value corresponding to the needs.

Further, the retrieval unit may retrieve training assistants with the index value higher than a predetermined threshold as the potential training assistants from the plurality of training assistants. It is thereby possible to identify training assistants who are more likely to meet the needs.

Further, the track record information may contain physical ability information before and after recovery of trainees to whom the assistance has been provided in the past and a recovery period, and the index value calculation unit may calculate, as the index value, recovery efficiency of each of the training assistants based on the physical ability information before and after recovery and the recovery period. It is thereby possible to more accurately represent the assisting ability of each training assistant by an index based on the past recovery efficiency.

Alternatively, the track record information may contain recovery efficiency of physical ability of trainees to whom the assistance has been provided in the past, and the index value calculation unit may perform the statistical processing of the recovery efficiency of each of the potential training assistants. It is thereby possible to more accurately evaluate facilities with regard to the needs based on the recovery efficiency of a potential training assistant who belongs to each facility.

Further, the output unit may output the list of facilities where the facilities are divided by area. It is thereby possible to easily know rehabilitation facilities in an area desired by a trainee.

Alternatively, the retrieval apparatus may include an index value calculation unit configured to calculate, for each of the training assistants, an index value of assisting ability related to the needs from the track record information, and the retrieval unit may retrieve training assistants with the index value higher than a predetermined threshold as the potential training assistants from the plurality of training assistants. It is thereby possible to appropriately identify training assistants who are more likely to meet the needs.

In this case, the output unit may assigns levels to the plurality of potential training assistants according to the index values, and output an assigned level to each of the potential training assistants. The training assistants can thereby know at which level his/her track record of rehabilitation is ranked among other training assistants with regard to the needs of rehabilitation. This contributes to the self-development of the training assistants in the rehabilitation capability (assisting ability).

The retrieval apparatus may further include an acquisition unit configured to acquire the track record information from an outside. It is thereby possible to easily collect a larger quantity of track record information from a wide range.

Alternatively, the retrieval apparatus may further include a storage unit configured to store definition information associating a cluster of the training assistants classified by cluster analysis of the track record information with the needs, and the retrieval unit may retrieve, from the definition information, a cluster of the training assistants associated with the needs as the plurality of potential training assistants. In this manner, by retrieving the training assistant cluster that meets the needs with use of the predetermined definition information, it is possible to increase the speed and efficiency of processing of identifying potential training assistants who suite the needs at the time of retrieval.

In this case, the storage unit may further store facility information for performing the training, indicating a facility to which each of the plurality of training assistants belongs, and an index value of assisting ability related to the needs, of each of the training assistants classified into a cluster of the training assistants, the retrieval unit may identify a facility to which each of the plurality of potential training assistants belongs by referring to the facility information, the retrieval apparatus may further include an evaluation value calculation unit configured to calculate an evaluation value for each facility by performing predetermined statistical processing of the index value of each of the potential training assistants, and the output unit may output a list of facilities indicated by the identified facility in order of the evaluation value. It is thereby possible to calculate the evaluation value of each facility with use of the index value in the training assistant cluster. It is thus possible to further increase the speed and efficiency of processing of identifying potential training assistants who suite the needs at the time of retrieval.

Further, the receiving unit may further receive current physical ability information of the trainee, and the retrieval unit may retrieve a plurality of potential training assistants who meet the needs by further taking into consideration the received physical ability information. It is thereby possible to identify more appropriate training assistants according to the degree of recovery of the current physical ability of a trainee.

Further, the track record information may contain at least some of a measured value in a training device used for the training, physical ability information before and after training of the trainee, a detection value in the training device with regard to assistance by the training assistant, information about details of rehabilitation, and evaluation of the training assistant by the trainee. It is thereby possible to make a more accurate search.

A retrieval system according to a second aspect of the present disclosure includes a receiving unit configured to receive needs regarding training to be performed by a trainee to restore or maintain his/her physical ability, a retrieval unit configured to retrieve a plurality of potential training assistants who meet the needs based on track record information of a plurality of training assistants with regard to assistance in the training, and an output unit configured to output information based on the plurality of potential training assistants.

A retrieval method according to a third aspect of the present disclosure includes, in a computer, receiving needs regarding training to be performed by a trainee to restore or maintain his/her physical ability, retrieving a plurality of potential training assistants who meet the needs based on track record information of a plurality of training assistants with regard to assistance in the training, and outputting information based on the plurality of potential training assistants.

A retrieval program according to a fourth aspect of the present disclosure causes a computer to execute receiving processing of receiving needs regarding training to be performed by a trainee to restore or maintain his/her physical ability, retrieval processing of retrieving a plurality of potential training assistants who meet the needs based on track record information of a plurality of training assistants with regard to assistance in the training, and output processing of outputting information based on the plurality of potential training assistants.

According to the second, third and fourth aspects also, the same effects as in the first aspect can be obtained.

According to the present disclosure, it is possible to provide a retrieval apparatus, system, method, and program for retrieving a rehabilitation training assistant or a rehabilitation facility to which the training assistant belongs in order for a trainee to effectively undergo rehabilitation.

The above and other objects, features and advantages of the present disclosure will become more fully understood from the detailed description given hereinbelow and the accompanying drawings which are given by way of illustration only, and thus are not to be considered as limiting the present disclosure.

### BRIEF DESCRIPTION OF DRAWINGS

Fig. 1 is an overall conceptual view showing a configuration example of a rehabilitation support system according to a first embodiment;
Fig. 2 is a schematic perspective view showing a configuration example of a walking assist device in the rehabilitation support system of Fig. 1;
Fig. 3 is a block diagram showing a system configuration example of a gait training device in the rehabilitation support system of Fig. 1;
Fig. 4 is a block diagram showing the overall configuration of a retrieval system according to the first embodiment;
Fig. 5 is a block diagram showing the configuration of a retrieval apparatus according to the first embodiment;
Fig. 6 is a flowchart showing the flow of a retrieval method according to the first embodiment;
Fig. 7 is a block diagram showing the configuration of a retrieval apparatus according to a second embodiment;
Fig. 8 is a flowchart showing the flow of a retrieval method according to the second embodiment;
Fig. 9 is a block diagram showing the configuration of a retrieval apparatus according to a third embodiment;
Fig. 10 is a view showing an example of clusters of training assistants classified by cluster analysis of track record information; and
Fig. 11 is a flowchart showing the flow of a retrieval method according to the third embodiment.

### DESCRIPTION OF EMBODIMENTS

Specific embodiments of the present disclosure including the above-described aspects will be described hereinafter in detail with reference to the drawings. The same or corresponding elements are denoted by the same symbols throughout the drawings, and duplicated explanations are omitted as necessary for the sake of clarity.

### <First Embodiment>

A first embodiment will be described hereinafter with reference to the drawings.

### [System Configuration]

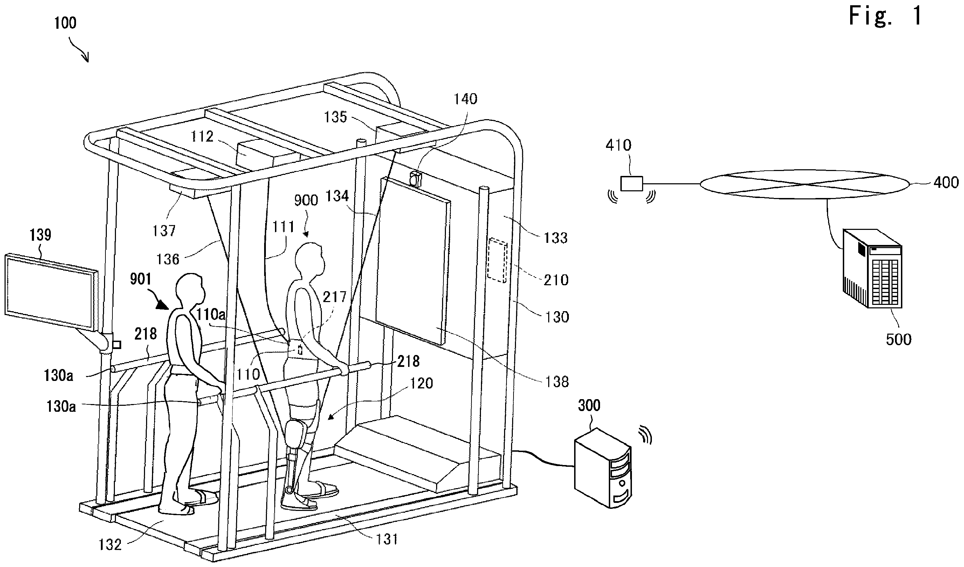

Fig. 1 is an overall conceptual view showing a configuration example of a rehabilitation support system according to a first embodiment. The rehabilitation support system (rehabilitation system) according to this embodiment is composed mainly of a gait training device 100, an external communication device 300, and a server (server device) 500.

The gait training device 100 is a specific example of a rehabilitation support device that supports the rehabilitation of a trainee (user) 900. The gait training device 100 is a device for the trainee 900 who is a hemiplegic patient whose one leg is paralyzed to undergo gait training under instruction of a training staff 901. The training staff 901 may be a therapist (physical therapist) or a doctor, and they may be also called a training instructor, a training helper, a training training assistant, a training assistant, and so on because they assists a trainee by instructions or helping.

The gait training device 100 mainly includes a control panel 133 mounted to a frame 130 that forms an overall framework, a treadmill 131 for the trainee 900 to walk on, and a walking assist device 120 to be worn on an affected leg, which is a paralyzed leg of the trainee 900.

The frame 130 is mounted vertically to the treadmill 131 installed on a floor. The treadmill 131 rotates a ring-shaped belt 132 by a motor, which is not shown. The treadmill 131 is a device that allows the trainee 900 to walk, and the trainee 900 who undergoes gait training steps on the belt 132 and tries to walk as the belt 132 moves. While the training staff 901 may stand on the belt 132 on the back of the trainee 900 and walk together as shown in Fig. 1, for example, the staff may be generally at the position where they can easily help the trainee 900, such as placing his/her feet on both sides of the belt 132.

The frame 130 supports the control panel 133 that contains an overall control unit 210 that controls a motor and a sensor, a training monitor 138, such as a liquid crystal panel, for example, that presents the progress of training, etc. to the trainee 900, and so on. Further, the frame 130 supports a front pulling unit 135 at the front overhead of the trainee 900, supports a harness pulling unit 112 at the overhead, and supports a rear pulling unit 137 at the rear overhead. The frame 130 includes handrails 130a for the trainee 900 to hold.

The handrails 130a are placed on both sides (left and right) of the trainee 900. Each of the handrails 130a is placed in parallel to the walking direction of the trainee 900. The handrails 130a can be adjusted up and down, and left and right. Thus, the handrails 130a may include a mechanism to change his/her height. Further, the handrails 130a may be configured to be able to change his/her slope angle by adjusting the handrail height to be different between the front and the back in the walking direction, for example. For example, the handrails 130a may slope in such a way that they gradually become higher toward the walking direction.

Further, the handrails 130a are equipped with a handrail sensor 218 that senses the load received from the trainee 900. For example, the handrail sensor 218 may be a pressure sensor sheet of resistance change sensing type where electrodes are arranged in matrix. Alternatively, the handrail sensor 218 may be a 6-axis sensor that combines a 3-axis acceleration sensor (x, y, z) and a 3-axis gyroscope sensor (roll, pitch, yaw). Note that, however, the type and the installation position of the handrail sensor 218 are not particularly limited.

A camera 140 functions as an imaging unit for observing the whole body of the trainee 900. The camera 140 is placed to face the trainee in close proximity to the training monitor 138. The camera 140 takes still images and videos of the trainee 900. The camera 140 includes a set of a lens and an image sensor that gives an angle of view wide enough to capture the whole body of the trainee 900 undergoing training. The image sensor is a CMOS (Complementary Metal-Oxide-Semiconductor) image sensor, for example, and it converts an optical image formed on an imaging plane into an image signal.

The front pulling unit 135 and the rear pulling unit 137 operate in collaboration to cancel the weight of the walking assist device 120 out to prevent the weight from causing a burden on the affected leg, and further assist in the forward swing of the affected leg in accordance with a set level.

One end of a front wire 134 is joined to a winding structure of the front pulling unit 135, and the other end is joined to the walking assist device 120. The winding structure of the front pulling unit 135 winds up or draws out the front wire 134 in accordance with the motion of the affected leg by turning on and off a motor, which is not shown. Likewise, one end of a rear wire 136 is joined to a winding structure of the rear pulling unit 137, and the other end is joined to the walking assist device 120. The winding structure of the rear pulling unit 137 winds up or draws out the rear wire 136 in accordance with the motion of the affected leg by turning on and off a motor, which is not shown. Such cooperative operation of the front pulling unit 135 and the rear pulling unit 137 cancels the weight of the walking assist device 120 out to prevent the weight from causing a burden on the affected leg and further assists in the forward swing of the affected leg in accordance with a set level.

For example, the training staff 901, acting as an operator, sets the assist level to high for a trainee who is suffering from severe paralysis. When the assist level is set high, the front pulling unit 135 winds up the front wire 134 with a relatively strong force at the timing of the forward swing. Once the training has progressed and assistance is no longer needed, the training staff 901 sets the assist level to minimum. When the assist level is set to minimum, the front pulling unit 135 winds up the front wire 134 only with a force enough to cancel out the weight of the walking assist device 120 at the timing of the forward swing of the affected leg.

The gait training device 100 includes a fall-prevention harness device as a safety device, which is composed mainly of a harness 110, a harness wire 111, and the harness pulling unit 112. The harness 110 is a belt to be worn around the waist of the trainee 900, and it is fixed to the waist with a hook and loop fastener, for example. The harness 110 has a joint hook 110a for joining one end of the harness wire 111, which is a hanging tool, and it can be referred to as a hanger belt. The trainee 900 wears the harness 110 in such a way that the joint hook 110a comes to the back.

One end of the harness wire 111 is joined to the joint hook 110a of the harness 110, and the other end is joined to a winding structure of the harness pulling unit 112. The winding structure of the harness pulling unit 112 winds up or draws out the harness wire 111 by turning on and off a motor, which is not shown. In such a structure, when the trainee 900 is about to fall, the fall-prevention harness device winds up the harness wire 111 in response to an instruction from the overall control unit 210 that has detected this motion, and thereby supports the upper body of the trainee 900 by the harness 110 to prevent the trainee 900 from falling.

The harness 110 includes a posture sensor 217 for detecting the posture of the trainee 900. The posture sensor 217 is a combination of a gyroscope sensor and an acceleration sensor, for example, and it outputs the tilt angle of the waist around which the harness 110 is worn with respect to the direction of a gravitational force.

A management monitor 139, which is mounted to the frame 130, is a display input device that is mainly used for monitoring and operation by the training staff 901. The management monitor 139 is a liquid crystal panel, for example, which has a touch panel on its surface. The management monitor 139 displays menu items related to training settings, parameter values during training, training results, and so on.

The walking assist device 120 is worn on the affected leg of the trainee 900, and assists the trainee 900 in walking by reducing the stress on the knee joint of the affected leg caused by flexion and extension. The walking assist device 120 includes a sensor, etc. for measuring the load on the sole of the foot, and outputs various data related to the forward movement of the leg to the overall control unit 210. Further, the harness 110 may be connected to the walking assist device 120 by using a connecting member (which is referred to hereinafter as a hip joint) having a rotating part. The details of the walking assist device 120 are described later.

The overall control unit 210 generates rehabilitation data that may contain configuration parameters related to training settings, various data related to the forward movement of the leg output as training results from the walking assist device 120, and so on. The rehabilitation data may contain data indicating the training staff 901 and his/her years of experience, skill level, and so on, data indicating a symptom, walking ability, recovery level, and so on of the trainee 900, various data output from a sensor, etc. installed outside the walking assist device 120, and so on. The details of the rehabilitation data are described later.

The external communication device 300 is one specific example of a transmitting means for transmitting the rehabilitation data to the outside. The external communication device 300 may have a function of receiving and temporarily storing the rehabilitation data output from the gait training device 100 and a function of transmitting the stored rehabilitation data to the server 500.

The external communication device 300 is connected to the control panel 133 of the gait training device 100 by an USB (Universal Serial Bus) cable, for example. Further, the external communication device 300 is connected to a network 400, such as the Internet or an intranet, through a wireless communication device 410 by a wireless LAN (Local Area Network), for example. Note that the gait training device 100 may include a communication device rather than the external communication device 300.

The server 500 is one specific example of a storage means for storing the rehabilitation data. The server 500 is connected to the network 400, and it has a function of accumulating the rehabilitation data received from the external communication device 300. The functions of the server 500 are described later.

Although the gait training device 100 is described as one example of the rehabilitation support device in the first embodiment, the rehabilitation support device is not limited thereto but may be any rehabilitation support device that supports the rehabilitation of a trainee. For example, the rehabilitation support device may be an upper limb rehabilitation support device that supports the rehabilitation of the shoulder and arm. Alternatively, the rehabilitation support device may be a rehabilitation support device that supports the rehabilitation of the balance ability of a trainee.

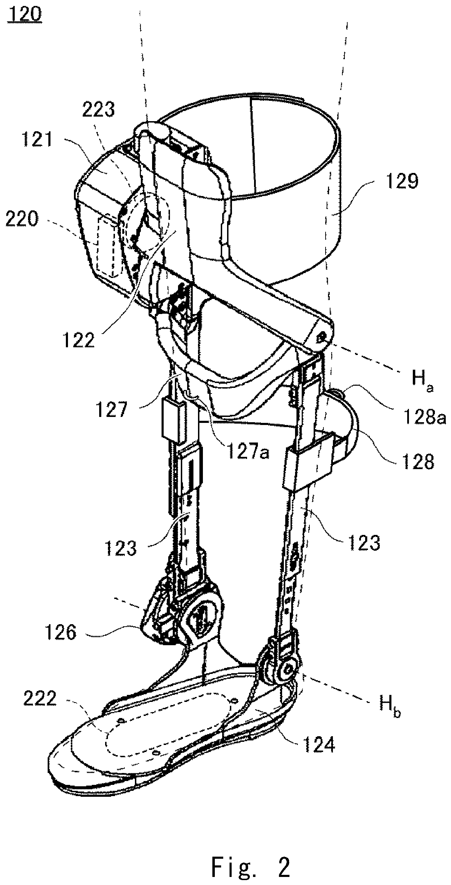

The walking assist device 120 is described hereinafter with reference to Fig. 2. Fig. 2 is a schematic perspective view showing a configuration example of the walking assist device 120. The walking assist device 120 mainly includes a control unit 121, a plurality of frames that support different parts of the affected leg, and a load sensor 222 for detecting the load on the sole of the foot.

The control unit 121 includes an assist control unit 220 that controls the walking assist device 120 and further includes a motor, which is not shown, that generates a drive force for assisting in the flexion and extension of the knee joint. The frames that support different parts of the affected leg include an upper leg frame 122 and lower leg frames 123 rotatably joined to the upper leg frame 122. Further, the frames include a foot frame 124 rotatably joined to the lower leg frames 123, a front joint frame 127 for joining the front wire 134, and a rear joint frame 128 for joining the rear wire 136.

The upper leg frame 122 and the lower leg frames 123 relatively rotate around a hinge shaft Ha, which is shown in the figure. The motor of the control unit 121 rotates in response to an instruction from the assist control unit 220 and thereby urges the upper leg frame 122 and the lower leg frames 123 to relatively open or close around the hinge shaft Ha. An angle sensor 223 included in the control unit 121 is a rotary encoder, for example, and it detects the angle between the upper leg frame 122 and the lower leg frames 123 around the hinge shaft Ha. The lower leg frames 123 and the foot frame 124 relatively rotate around a hinge shaft Hb, which is shown in the figure. The angular range of the relative rotation is adjusted in advance by an adjustment mechanism 126.

The front joint frame 127 is placed to extend horizontally on the front side of the upper leg and connect to the upper leg frame 122 on both sides. Further, a joint hook 127a for joining the front wire 134 is placed at the center of the width of the front joint frame 127. The rear joint frame 128 is placed to extend horizontally on the back side of the lower leg and connect to the lower leg frames 123 extending vertically on both sides. Further, a joint hook 128a for joining the rear wire 136 is placed at the center of the width of the rear joint frame 128.

The upper leg frame 122 includes an upper leg belt 129. The upper leg belt 129 is a belt that is formed integrally with the upper leg frame, and it is worn around the upper part of the affected leg to fix the upper leg frame 122 to the upper leg. This prevents the whole part of the walking assist device 120 from coming out of place on the leg of the trainee 900.

The load sensor 222 is a pressure sensor embedded in the foot frame 124. The load sensor 222 detects the amount and distribution of the vertical load on the foot sole of the trainee 900, and it may be configured to detect the center of pressure (COP), for example. The load sensor 222 is a pressure sensor sheet of resistance change sensing type where electrodes are arranged in matrix, for example.

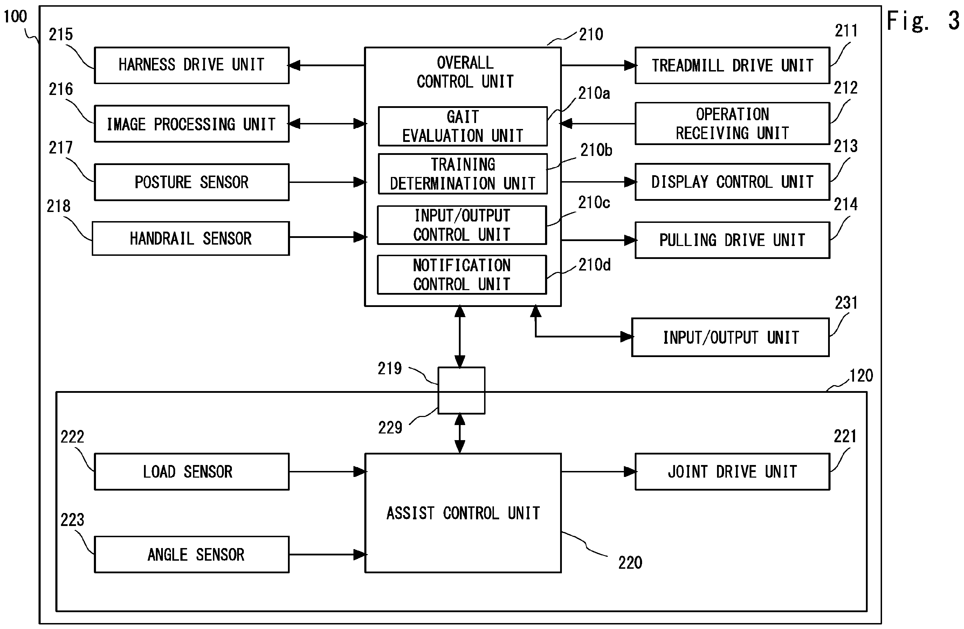

A system configuration example of the gait training device 100 is described hereinafter with reference to Fig. 3. Fig. 3 is a block diagram showing a system configuration example of the gait training device 100. As shown in Fig. 3, the gait training device 100 may include an overall control unit 210, a treadmill drive unit 211, an operation receiving unit 212, a display control unit 213, and a pulling drive unit 214. The gait training device 100 may further include a harness drive unit 215, an image processing unit 216, a posture sensor 217, a handrail sensor 218, a communication connection interface (IF) 219, an input/output unit 231, and a walking assist device 120.

The overall control unit 210 is an MPU (Micro Processing Unit), for example, and executes a control program read from a system memory and thereby exercises control over the whole device. The overall control unit 210 may include a gait evaluation unit 210a, a training determination unit 210b, an input/output control unit 210c, and a notification control unit 210d, which are described later.

The treadmill drive unit 211 includes a motor that rotates the belt 132 and its drive circuit. The overall control unit 210 sends a drive signal to the treadmill drive unit 211 and thereby carries out rotation control of the belt 132. The overall control unit 210 adjusts the rotation speed of the belt 132 in accordance with the walking speed set by the training staff 901, for example.

The operation receiving unit 212 receives input operation from the training staff 901 and transmits an operation signal to the overall control unit 210. The training staff 901 uses operation buttons on the device, a touch panel superimposed on the management monitor 139, an accompanying remote control, and so on, which constitute the operation receiving unit 212. By this operation, it is possible to give a command to turn power on/off or start training, input a value related to setting, and select a menu item. Note that the operation receiving unit 212 may receive input operation from the trainee 900.

The display control unit 213 receives a display signal from the overall control unit 210 and generates a display image, and displays this image on the training monitor 138 or the management monitor 139. The display control unit 213 generates an image showing the progress of training or a real time video captured by the camera 140 in accordance with the display signal.

The pulling drive unit 214 includes a motor for pulling the front wire 134 and its drive circuit, which constitute the front pulling unit 135, and a motor for pulling the rear wire 136 and its drive circuit, which constitute the rear pulling unit 137. The overall control unit 210 sends a drive signal to the pulling drive unit 214 and thereby controls the winding of the front wire 134 and the winding of the rear wire 136. In addition to the winding operation, the overall control unit 210 controls the drive torque of the motor and thereby controls the pulling force of each wire. The overall control unit 210 identifies the timing when the affected leg switches from stance to swing from a detection result of the load sensor 222 and increases or decreases the pulling force of each wire in synchronization with this timing, for example, and thereby assists in the forward swing of the affected leg.

The harness drive unit 215 includes a motor for pulling the harness wire 111 and its drive circuit, which constitute the harness pulling unit 112. The overall control unit 210 sends a drive signal to the harness drive unit 215 and thereby controls the winding of the harness wire 111 and the pulling force of the harness wire 111. The overall control unit 210 winds up the harness wire 111 to a certain point when it predicts the falling of the trainee 900, for example, and thereby protects the trainee from falling.

The image processing unit 216 is connected to the camera 140 and can receive an image signal from the camera 140. In response to an instruction from the overall control unit 210, the image processing unit 216 receives an image signal from the camera 140, performs image processing of the received image signal and generates image data. Further, in response to an instruction from the overall control unit 210, the image processing unit 216 may perform image processing of the image signal received from the camera 140 and carries out specific image analysis. For example, the image processing unit 216 detects the position (stance position) of the affected leg in contact with the treadmill 131 by image analysis. To be specific, the image processing unit 216 calculates the stance position by extracting an image region near the end of the foot frame 124 and analyzing an identification marker put on the belt 132 overlapping this end, for example.

The posture sensor 217 detects the tilt angle of the waist of the trainee 900 with respect to the direction of a gravitational force as described above, and transmits a detection signal to the overall control unit 210. Using the detection signal from the posture sensor 217, the overall control unit 210 calculates the posture of the trainee 900, which is, the tilt angle of the trunk to be specific. Note that the overall control unit 210 and the posture sensor 217 may be connected by wire or connected by near field communication.

The handrail sensor 218 detects the load on the handrails 130a. Specifically, the load placed because the trainee 900 cannot bear his/her own body weight with the legs is applied to the handrails 130a. The handrail sensor 218 detects this load and transmits a detection signal to the overall control unit 210.

The overall control unit 210 serves also as a function execution unit that executes control and various calculations related to control. The gait evaluation unit 210a evaluates whether the walking motion of the trainee 900 is abnormal gait or not by using the data acquired from the sensors. The training determination unit 210b determines a training result of a series of walking training based on a cumulative number of times the abnormal gait is detected which is evaluated by the gait evaluation unit 210a, for example. The overall control unit 210 may generate this determination result or the cumulative number of times of abnormal gait, based on which the result is determined, as a part of the rehabilitation data.

Note that a method of determination, including criteria for this determination, is not limited. For example, the determination may be made by comparing the amount of the motion of the paralyzed part of the body with a reference value for each gait phase. The gait phase is each phase of one gait cycle of the affected leg (or the non-affected leg) which is classified as a stance phase in a stance position, a transition phase from the stance phase to a swing phase in a swing position, the swing phase, a transition phase from the swing phase to the stance phase, and so on. The gait phase can be classified (determined) based on a detection result of the load sensor 222 as described above, for example. Although one gait cycle may be made up of the stance phase, the transition phase, the swing phase and the transition phase as described above, any phase can be the initial phase of the cycle. Alternatively, one gait cycle may be made up of double support, single (affected leg) support, double support, and single (non-affected leg) support, for example, and any phase can be the initial phase of the cycle in this case also.

Further, the gait cycle focusing on the right leg or the left leg (the non-affected leg or the affected leg) may be classified into more detailed periods. For example, the stance phase may be divided into initial contact and four periods, and the swing phase may be divided into three periods. The initial contact is a moment when the foot observed comes into contact with the floor, and the four periods in the stance phase are: loading response, mid-stance, terminal stance, and pre-swing. The loading response is a period from initial contact to a moment when the opposite foot leaves the floor (opposite foot-off). The mid-stance is a period from opposite foot-off to a moment when the heel of the foot observed leaves the floor (heel-off). The terminal stance is a period from heel-off to opposite initial contact. The pre-swing is a period from opposite initial contact to when the foot observed leaves the floor (foot-off). The three periods in the swing phase are: initial swing, mid-swing, and terminal swing. The initial swing is a period from the end of the pre-swing (foot-off) until both feet are next to each other (feet adjacent). The mid-swing is a period from feet adjacent until the tibia becomes vertical (tibia vertical). The terminal swing is a period from tibia vertical to the next initial contact.

The communication connection IF 219 is an interface connected to the overall control unit 210, and it is an interface for giving a command to the walking assist device 120 worn on the affected leg of the trainee 900 or receiving the sensor information.

The walking assist device 120 may include a communication connection IF 229 connected to the communication connection IF 219 by wired or wireless. The communication connection IF 229 is connected to the assist control unit 220 of the walking assist device 120. The communication connection IFs 219 and 229 are communication interfaces such as wired LAN or wireless LAN, for example, that comply with communication standards.

The walking assist device 120 may further include the assist control unit 220, a joint drive unit 221, a load sensor 222, and an angle sensor 223. The assist control unit 220 is an MPU, for example, and executes a control program read from the overall control unit 210 and thereby exercises control over the walking assist device 120. Further, the assist control unit 220 gives notification about the status of the walking assist device 120 to the overall control unit 210 through the communication connection IFs 219 and 229. In addition, the assist control unit 220 receives an instruction from the overall control unit 210 and performs control such as starting or stopping the walking assist device 120.

The joint drive unit 221 includes a motor of the control unit 121 and its drive circuit. The assist control unit 220 sends a drive signal to the joint drive unit 221 and thereby urges the upper leg frame 122 and the lower leg frames 123 to relatively open or close around the hinge shaft Ha. In this operation, it is possible to assist in knee flexion and extension and prevent the knee from giving away.

The load sensor 222 detects the amount and distribution of the vertical load on the foot sole of the trainee 900 as described above, and transmits a detection signal to the assist control unit 220. The assist control unit 220 receives and analyzes this detection signal and thereby determines whether it is in stance or swing, estimates switching and so on.

The angle sensor 223 detects the angle between the upper leg frame 122 and the lower leg frames 123 around the hinge shaft Ha as described above, and transmits a detection signal to the assist control unit 220. The assist control unit 220 receives this detection signal and calculates the knee joint opening angle.

The input/output unit 231 includes an USB (Universal Serial Bus) interface, for example, and it is a communication interface for connecting to external equipment (the external communication device 300 or other external equipment). The input/output control unit 210c of the overall control unit 210 communicates with external equipment through the input/output unit 231, and rewrites a control program in the overall control unit 210 or a control program in the assist control unit 220, receives commands, outputs generated rehabilitation data, and so on. The gait training device 100 communicates with the server 500 through the input/output unit 231 and the external communication device 300 by control of the input/output control unit 210c. For example, the input/output control unit 210c can make control of transmitting the rehabilitation data to the server 500 or control of receiving a command from the server 500 through the input/output unit 231 and the external communication device 300.

The notification control unit 210d controls the display control unit 213 or a separate audio control unit, etc. when the need to give notification to the training staff 901 arises and thereby makes notification using the management monitor 139 or a separate speaker. The details of this notification are described later. The case where the need to give notification to the training staff 901 arises may be when a command for giving notification is received from the server 500.

The details of the server 500 are described hereinafter.

As described above, the gait training device 100 transmits various rehabilitation data to the server 500 through the external communication device 300. The server 500 may be configured to receive the rehabilitation data from a plurality of gait training devices 100, so that it can receive a large quantity of rehabilitation data. The server 500 is a processing device that processes various types of data.

### [Rehabilitation Data]

Data which the server 500 according to this embodiment can collect from the gait training device 100, etc. is collectively referred to as rehabilitation data, which is described hereinbelow. The rehabilitation data mainly includes: (1) configuration parameters of the gait training device 100, (2) detection data detected by a sensor, etc. in the gait training device 100, (3) data related to the trainee 900, and (4) data related to the training staff 901. The rehabilitation data of the above-described (1) to (4) may be collected in association with date and time of acquisition. Further, the detection data or the configuration parameters may be collected as time-series log data, or they may be a feature quantity, etc. extracted for data of every certain period of time.

The rehabilitation data is data obtained mainly by operation input, automatic input, measurement by a sensor, and so on in the gait training device 100. Further, the rehabilitation data may include video data captured by the camera 140. The rehabilitation data may be data for each rehabilitation training date, and in this case, it may be referred to daily report data. The following description is based on the assumption that the server 500 collects the rehabilitation data generated by the gait training device 100; however, the server 500 may acquire a part of the rehabilitation data from another server, for example, different from the gait training device 100. For example, a part of the rehabilitation data may be the details of data of the above-described (3) such as a symptom of the trainee 900, the details of data of the above-described (4) such as years of experience of a physical therapist (PT), and so on. The former data may be stored in another server as health record information of the trainee 900, and the latter data may be stored in another server as a PT resume, etc. Note that, because the training staff 901 is a physical therapist in many cases, a training assistant 11 is sometimes referred to simply as "PT" in the following description.

The server 500 may receive the rehabilitation data from the gait training device 100 upon generation of the rehabilitation data, or at regular intervals such as daily or weekly. Alternatively, the server 500 may receive the rehabilitation data from the gait training device 100 at the start of training and further receive any of the data of the above-described (1) and (2) for which a change is made during training. Any of the gait training device 100 and the serve 500 may take the initiative to transmit and receive the rehabilitation data.

The above-described (1) is described hereinafter.

The data of (1) may be defined, together with the detection data of (2), as training data of the trainee 900 acquired in the course of rehabilitation using the gait training device 100.

The configuration parameters of the gait training device 100 are data entered by an operator or set automatically in order to configure the operation of the gait training device 100, for example. As described above, an operator is generally the training staff 901 who actually attends the training of the trainee 900, and the following description is based on the assumption that the operator is the training staff 901.

The gait training device 100 allows adjustment of the difficulty of gait training by the configuration parameters. The configuration parameters may include a parameter indicating the level of difficulty and, in this case, some or all of the other configuration parameters can be changed in accordance with a change in this level. The training staff 901 raises the difficulty of gait training as the recovery of the trainee 900 progresses. In other words, the training staff 901 reduces the assistance by the gait training device 100 as the walking ability of the trainee 900 increases. On the other hand, the training staff 901 increases the assistance when an abnormality occurs during the gait training. By appropriately adjusting the configuration parameters by the training staff 901, the trainee 900 can carry out appropriate gait training and can thereby undergo rehabilitation efficiently.

Specific examples of configuration parameters are as follows.

The configuration parameters may be partial weight-bearing [%], a position along the vertical direction of the handrails 130a [cm], a position along the horizontal direction of the handrails 130a [cm], the presence or absence of a hip joint, limitation of ankle joint plantarflexion [deg], limitation of ankle joint dorsiflexion [deg], and so on, for example. Further, the configuration parameters may be a treadmill speed [km/h], forward swing assistance [level], and a ratio between before and after forward swing [before/after]. Furthermore, the configuration parameters may be knee extension assistance [level], a knee flexion angle [deg], a knee bending time [sec], a raise height [mm], an unweighting threshold [%], and a load threshold [%], for example. Data contained in the rehabilitation data, including the configuration parameters described above, may be in any units.

The partial weight-bearing is the percentage of relieving the bearing of the weight of the trainee 900 by pulling the harness wire 111 by the harness pulling unit 112. The training staff 901 sets the partial weight-bearing to a smaller value as the difficulty of intended gait training increases. The position along the vertical direction and the position along the horizontal direction of the handrails 130a is the amount of adjustment from a reference position of the handrails 130a. The presence or absence of a hip joint is whether or not a hip joint is attached or not. The limitation of ankle joint plantarflexion and the limitation of ankle joint dorsiflexion define the angular range in which the lower leg frame 123 and the foot frame 124 can rotate around the hinge shaft Hb. The limitation of ankle joint plantarflexion corresponds to the upper limit angle on the front side, and the limitation of ankle joint dorsiflexion corresponds to the maximum angle on the back side. Thus, the limitation of ankle joint plantarflexion and the limitation of ankle joint dorsiflexion are limit values of the angle of bending the ankle joint to lower the toe and to raise the toe, respectively. The training staff 901 sets the values of the limitation of ankle joint plantarflexion and the limitation of ankle joint dorsiflexion so as to increase the angular range as the difficulty of intended gait training increases.

The treadmill speed is the walking speed on the treadmill 131. The training staff 901 sets the treadmill speed to higher as the difficulty of intended gait training increases. The forward swing assistance is a level in accordance with the pulling force applied by the front wire 134 upon forward swing of the leg, and as this level increases, the maximum pulling force increases. The training staff 901 sets the forward swing assistance to a lower level as the difficulty of intended gait training increases. The before-and-after forward swing ratio is a ratio of the pulling force by the front wire 134 and the pulling force by the rear wire 136 upon forward swing of the leg.

The knee extension assistance is a level in accordance with the drive torque of the joint drive unit 221, which is applied to prevent the knee from giving away during the stance, and as this level increases, the drive torque increases. The training staff 901 sets the knee extension assistance to a lower level as the difficulty of intended gait training increases. The knee flexion angle is an angle when performing the knee extension assistance. The knee bending time is a period of time during which knee extension assistance is provided, and when its value is large, assistance is given to bend the knee slowly, and when its value is small, assistance is given to bend the knee quickly.

The raise height is the height of a member such as a cushion that is attached to the shoe sole of the opposite leg to the paralyzed leg (which is the leg opposite to the leg with the walking assist device 120, which is an assistive device). The unweighting threshold is one of thresholds for the load on the sole of the foot, and when the load falls below this threshold, the forward swing assistance is disabled. The load threshold is one of thresholds for the load on the sole of the foot and when the load exceeds this threshold, the forward swing assistance is enabled. In this manner, the walking assist device 120 may be configured to be able to adjust the bending motion of the knee by four configuration parameters: the knee flexion angle, the knee bending time, the unweighting threshold, and the load threshold.

Further, the gait training device 100 may be configured to feed back the setting values of parameters such as a load and an angle, a target value, a target value achievement rate, target achievement timing, and so on by a sound through a speaker, which is not shown, for example. The above-described configuration parameters may include parameters for setting such as whether or not to play a feedback sound and the volume of a sound.

The above-described configuration parameters may be different from configuration parameters directly related to training. For example, the above-described configuration parameters may be setting values for an image, music, a game type, game difficulty, and so on to be provided by the training monitor 138 or a speaker, which is not shown, in order to enhance the motivation of the trainee 900.

Note that the above-described configuration parameters are just examples, and other configuration parameters may be used. Alternatively, some of the above-described configuration parameters may be not used. Further, although most of the above configuration parameters are parameters for adjusting the difficulty of training as described above, parameters not related to the difficulty may be included. For example, the gait training device 100 may be configured to display an attention-attracting icon image to be displayed on the training monitor 138. The configuration parameters not related to the difficulty may be parameters for improving the concentration of the trainee 900 on training, such as the size and the display interval of such an attention-attracting icon image, for example. Furthermore, time information such as the date and time when they are set or timing information different from time (for example, information for distinguishing between the stance phase, the swing phase, and so on in one gait cycle) may be added to the above-described configuration parameters.

The above-described (2) is described hereinafter.

The detection data of (2) may be defined, together with the data of (1), as training data of the trainee 900 acquired in the course of rehabilitation using the gait training device 100.

The detection data is, mainly, sensor data. The sensor data is sensor values detected by the sensors in the gait training device 100. For example, the sensor data may be the tilt angle of the trunk detected by the posture sensor 217, the load or the slope angle detected by the handrail sensor 218, the angle detected by the angle sensor 223, and so on. The sensors that output the sensor data are an acceleration sensor, an angular velocity sensor, a position sensor, a light sensor, a torque sensor, a weight sensor, and so on. Further, an encoder included in a motor of a winding structure, etc. for the front wire 134, the rear wire 136 and the harness wire 111 may be used as a sensor. Furthermore, a torque sensor (load cell) of a motor may be used as a sensor, or a current detector that detects a drive current value for driving the motor may be used as a sensor.

The sensor data may contain line-of-sight data acquired by a line-of-sight sensor that detects the line of sight, for example. Such line-of-sight data may be also obtained by detecting the line of sight from image processing based on an image at least showing the eyes of the trainee 900 or obtained by determining the orientation (looking up, looking down, etc.) of the face based on an image at least showing the face of the trainee 900. Such data may be contained in the above-described detection data. Further, the detection data may be voice data acquired by a voice acquisition unit such as a microphone that acquires the voice of the trainee 900 or the training staff 901, text data obtained by analyzing this voice data, or data obtained by analyzing this text data. The voice of the training staff 901 may contain the voice talked to the trainee 900 for correcting the gait, etc. Furthermore, the sensor data may be detection data of the brain wave of the trainee 900 detected by an electroencephalograph, or detection data of the brain wave of the training staff 901 detected by an electroencephalograph.

While the line-of-sight sensor, an imaging unit that takes the above-described image, the microphone and so on may be mounted on the main body of the training device 100, they may be mounted on a glasses-type wearable terminal to be worn by the trainee 900, for example. This terminal is equipped with a wireless communication unit that performs wireless communication of data by wireless communication technology such as Bluetooth (registered trademark), and the gait training device 100 may be also equipped with a wireless communication unit. The gait training device 100 can thereby acquire data acquired by the wearable terminal by wireless communication. The electroencephalograph may be mounted on the main body of the training device 100 and configured to be able to detect the brain wave of the trainee 900 and the brain wave of the training staff 901 separately from each other, though high detection accuracy is needed in this case. The electroencephalograph, however, is preferably mounted at the position where it comes into close proximity with a target person, such as on the above-described glasses-type wearable terminal (for example, on the temple of glasses).

Further, the detection unit, such as a sensor, that acquires the detection data is not limited to those described with reference to Figs. 1 to 3, the glasses-type wearable terminal described above, and so on. For example, the trainee 900 may wear clothing with a clothing-embedded biosensor and/or a clothing-embedded touch sensor. The clothing is not limited to upper body clothing, and they may be lower body clothing or a set of upper and lower body clothing, or one to be worn on a part of the body, such as the harness 110, for example. Further, clothing and the gait training device 100 are equipped with a wireless communication unit as described above. The gait training device 100 can thereby acquire data acquired by the clothing-embedded biosensor or the clothing-embedded touch sensor by wireless communication. The clothing-embedded biosensor can acquire vital data such as the heart rate of a person wearing it. The clothing-embedded touch sensor can acquire information where the trainee 900 who is wearing it is touched from the outside, which is data indicating information about the position where the training staff 901 touches the trainee 900.

The detection data may further contain values calculated based on detection signals from a plurality of sensors or statistics obtained from statistical processing of detection signals from one or a plurality of sensors and so on, not limited to values indicated by the detection signals detected by the sensors. The statistics may be various statistics such as the average value, the maximum value, the minimum value and the standard deviation value, for example, and they may be static statistics or dynamic statistics during a certain period of time such as one day, one training or one gait cycle, for example.

For example, the sensor data may contain the knee joint opening angle calculated from the angle between the upper leg frame 122 and the lower leg frame 123 detected by the angle sensor 223. Further, the sensor data regarding the angle sensor may contain the angular velocity obtained from the differentiation of the angle. The sensor data regarding the acceleration sensor may be the velocity obtained from the differentiation of the acceleration or the position obtained from performing the differentiation twice.

For example, the detection data may contain the average value, the total value, the maximum value, the minimum value and the representative value as follows for each day, or each rehabilitation training in one day. The average value may be the average speed (total walking distance/total walking time) [km/h], the average value of the stride length [cm], the walking rate [steps/min] indicating the number of steps per minute, the walking PCI [beat/m], fall prevention help [%], and so on. The average speed may be a value calculated from the speed set value of the treadmill 131 or a value calculated from a drive signal in the treadmill drive unit 211, for example. The stride length is the distance from ground contact of one heel to the next ground contact of the same heel. The PCI stands for Physiological Cost Index, and the walking PCI indicates the energy efficiency during walking. The fall prevention help [%] indicates the percentage that calculates the fall prevention helps [times] indicating the number of times the training staff 901 gives the fall prevention help for the trainee 900 per step, which is the percentage of performing fall prevention help per step.

The total value may be the walking time [seconds], the walking distance [m], the number of steps [steps], the fall prevention help [times], the body part and the number of times per body part [times] for which the fall prevention help is given, and so on.

The maximum value or the minimum value in this example may be the maximum value or the minimum value of the continuous walking time [seconds], the continuous walking distance [m], the successive number of steps [steps], and so on, the minimum value of the walking PCI [beat/m] (in other words, the longest distance the trainee can walk per beat), and so on. The representative value may be the value used most frequently as the speed of the treadmill 131 (representative speed [km/h]).

As described above, data supplied directly or indirectly from detection units such as sensors may be contained in the detection data. Further, time information such as the date and time when data is detected or timing information different from time may be added to the detection data.

Note that the above-described detection data are just examples, and other detection data may be used. Alternatively, some of the above-described detection data may be not used. Specifically, when the detection data is used as the rehabilitation data, the server 500 may collect one or more detection data.

The above-described (3) is described hereinafter.

The data related to the trainee 900 (which is referred to hereinafter as trainee data) indicates attributes, etc. of the trainee 900, for example. The trainee data may contain the age, the gender, the physical size (body height, weight, etc.), symptom information, Br. stage, SIAS, initial gait FIM, latest gait FIM, and so on of the trainee 900. The trainee data may also contain the name or ID of the trainee 900, and may further contain preference information indicating the preference of the trainee 900, personality information indicating the personality of the trainee 900, and so on. Further, the trainee data may contain, as FIM, exercise items different from those related to the ability to walk, and may also contain cognitive items. Thus, the trainee data may contain various data indicating the physical ability of the trainee 900. Note that a part or whole of the trainee data may be referred to as physical information, basic information, or trainee feature information, and so on.

The symptom information may contain information indicating an initial symptom, the time of emergence of the symptom, and the current symptom, and it can be considered that the trainee 900 is in need of rehabilitation mainly due to the symptom contained in this information. Note that, however, a symptom that is not likely to be directly related to the rehabilitation may be also contained in the symptom information. Further, the symptom information may contain the type of disease (disease name or disorder name) the trainee is affected, such as stroke (cerebrovascular disease) or spinal cord injury, and the affected area (the area of injury), and in some disease type, may contain his/her category. For example, stroke may be classified into cerebral infarction, intracranial hemorrhage (brain hemorrhage/subarachnoid hemorrhage), and so on.

The Br. stage, which indicates Brunnstrom Recovery stage, divides the process of recovery from hemiplegia into six stages based on observation. The trainee data may contain the items in the lower extremity section, which are main items related to the gait training device 100. The SIAS stands for Stroke Impairment Assessment Set, which is an index for comprehensively evaluating the dysfunction caused by a stroke. The SIAS may contain hip-flexion test (Hip-Flex), knee-extension test (Knee-Ext), and foot-pat test (Foot-Pat). The SIAS may also contain lower extremity light touch (Touch L/E), lower extremity position sense (Position L/E), abdominal muscle strength (Abdominal), and verticality test (Verticality).

The FIM (Functional Independence Measure) defines one of evaluation methods for ADL (Activities of Daily Life). In the FIM, each item is rated on a 7-point scale based on the level of assistance.

For example, the gait FIM is a universal index indicating the level of recovery. When a trainee can walk 50 m or longer without helper and harness (assistive device), the score is 7 points, which is highest. When, on the hand, the trainee can only walk less than 15 m with the assistance of one helper, the score is 1 points, which is lowest. When the trainee can move 50 m with the minimal assistance (the level of assistance = less than 25%), the score is 4 points, and when the trainee can move 50 m with the moderate assistance (the level of assistance = 25% or more), the score is 3 points. Thus, as the recovery progresses, the gait FIM of the trainee 900 increases accordingly.

As described above, the latest gait FIM employed in the gait training device 100 serves as an index indicating the physical ability of the trainee 900 and also as an index indicating the level of recovery of the trainee 900 from the start of rehabilitation. Thus, the gait FIM is an important index in terms of knowing the progress of the rehabilitation of the trainee 900. Further, the amount of change or the rate of change from the initial gait FIM to the latest gait FIM also serves as an index indicating the level of recovery. The rate of change may be referred to also as FIM efficiency, and it may be the value obtained by dividing the gain (the amount of change) of FIM up to the present by the number of days rehabilitation is performed, the number of days elapsed indicating the period of rehabilitation, or the length of hospital stay when the trainee 900 is a hospitalized patient, for example.

Further, the gait FIM may be regarded as a score under certain conditions at the time of evaluation, such as when adaptive equipment is worn, and in this case, information indicating the conditions applied at the time of evaluation may be added to information indicating the gait FIM. The conditions may contain a raise height, a harness used (for example, the walking assist device 120, another walking assist device, without a harness etc.), the setting of the angle of a knee or ankle part of this harness, etc. whether level-ground walking or slope walking, and so on, when this information is acquired. Further, the gait FIM is typically a gait FIM in level-ground walking, and level-ground walking information indicating it may contain information such as the longest distance (maximum continuous walking distance [m]) at the time of level ground walk evaluation.

As described above, the trainee data of the above-described (3) may contain index data containing at least one of the symptom, the physical ability and the recovery level of the trainee 900 regarding the rehabilitation performed by the trainee 900 by using the gait training device 100. The data, such as the latest gait FIM, that can be included in two concepts of the physical ability and the recovery level may be generally included in one of them; however, it may be included in both of them. The same applies to all items of the rehabilitation data, and data of a certain item can be treated as one or a plurality of the above-described data (1) to (4). Further, to the above-described trainee data, time information such as the date and time when the data is acquired, such as the date and time of measurement of the gait FIM, may be added.

The above-described (4) is described hereinafter.

The data related to the training staff 901 (which is referred to hereinafter as staff data) indicates attributes, etc. of the training staff 901, for example. The staff data may contain the name or ID, the age, the gender, the physical size (body height, weight, etc.), the name of the hospital to which they belong, the years of experience as a PT or doctor, and so on of the training staff 901. The staff data may contain a value indicating timing to assist the trainee 900 in numerical terms as data related to a helper.

In the case where a plurality of training staffs assist in rehabilitation at the same time, the rehabilitation data may contain the staff data of the plurality of staffs. Each staff data may contain information indicating whether the staff is a primary training staff or an assistant training staff. In addition or alternatively to such information, each staff data may contain information indicating whether the staff is a training staff who makes settings or check images on the management monitor 139 or a training staff who is there only to hold the trainee 900, and so on.

Further, the gait training device 100 may be configured to be able to input a rehabilitation plan for the trainee 900. Data of the input rehabilitation plan may be also contained as the staff data related to the training staff 901 who inputs the data, or as the rehabilitation data belonging to another category. Further, in order to deal with a change of the training staff 901, the gait training device 100 may be configured to be able to input notes and things to keep in mind when assisting in the training of this trainee 900 in the future. Data of this input data may be also contained as the staff data related to the training staff 901 who inputs the data, or as the rehabilitation data belonging to another category.

Such data is contained in the rehabilitation data because there can be a case where a certain training staff can successfully carry out the training of the trainee 900 with the presence of such notes from another skilled training staff. Further, to the staff data, time information such as the date and time when the data is input, such as the date and time of input of a rehabilitation plan, may be added.

The server 500 is an example of the retrieval apparatus according to this embodiment. Thus, the present embodiment is described hereinafter in terms of a retrieval system, mainly regarding the functions, configuration and operations of the retrieval apparatus according to this embodiment.

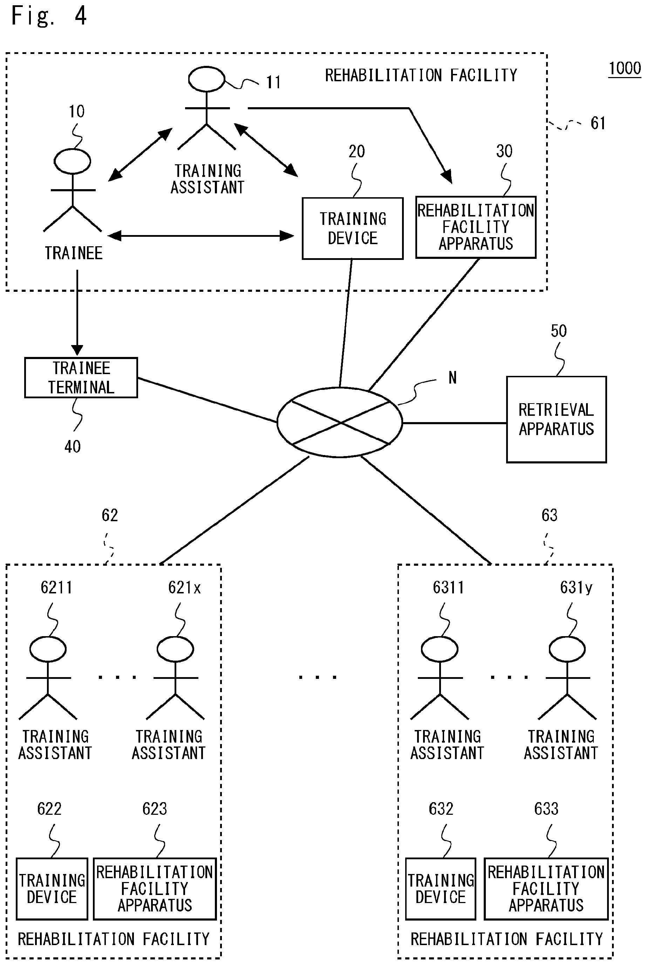

Fig. 4 is a block diagram showing the overall configuration of a retrieval system 1000 according to the first embodiment. The retrieval system 1000 is an information system that at least includes a retrieval apparatus 50, and the retrieval apparatus 50 is one or more information processing apparatuses that include the functions and configuration of the server 500 described above. The retrieval apparatus 50 is connected for communication through a network N with a training device 20, a rehabilitation facility apparatus 30, a trainee terminal 40, a training device 622, a rehabilitation facility apparatus 623, a training device 632, and a rehabilitation facility apparatus 633. The network N is a communication network such as the Internet, an intranet, a mobile telephone network or a LAN (Local Area Network).

A rehabilitation facility 61 is a facility, such as a medical institution, for a trainee 10 to undergo training with the assistance of a training assistant 11 in order to restore or maintain his/her physical ability. The rehabilitation facility 61 includes the training device 20 and the rehabilitation facility apparatus 30. The trainee 10 is a person equivalent to the above-described trainee 900, and, for example, the trainee 10 is a rehabilitation patient who undergoes rehabilitation in order to restore the physical ability after surgery performed because of suffering from a serious illness or a severe injury. Alternatively, the trainee 10 may be an elderly person, etc. who undergoes rehabilitation in order to maintain the physical ability. The training assistant 11 is a person equivalent to the above-described training staff 901 and generally assists in the rehabilitation of a plurality of trainees who stay in or visit the rehabilitation facility 61. In general, a plurality of training assistants belong to the rehabilitation facility 61. Further, there is a case where two or more training assistants assist one trainee at the same or different timing. The rehabilitation is a training process to be performed in order to restore or maintain the physical function or to reduce the physical disability, regardless of whether it is covered by insurance or not, or whether medical equipment is used or not.

The training device 20 is a device for the trainee 10 to undergo training with the assistance of the training assistant 11 by instructions or helping in order to restore or maintain his/her physical ability. The training device 20 may be a rehabilitation support device such as the gait training device 100 described above, for example, though not limited thereto.

In this case, it is assumed that the training device 20 acquires the whole or part of the rehabilitation data of the above-described (1) to (4) and stores them into an internal storage device. The trainee data of the above-described (3) may be associated with identification information of the training assistant 11 who has assisted in training. Alternatively, the staff data of the above-described (4) may be associated with identification information, etc. of the trainee 10 whom the training assistant 11 has assisted in training. Thus, information that associates the identification of the training assistant 11 with values of the Br. stage, the SIAS, the initial gait FIM or the latest gait FIM, among the trainee data of (3), before and after recovery (for example, FIM at the start of rehabilitation and FIM after rehabilitation (current FIM, latest FIM)), and a period of time required for recovery is one example of track record information of a plurality of training assistants with regard to the assistance of training. Alternatively, information that associates the identification of the training assistant 11 with symptoms for which each PT has assisted in training, body parts rehabilitated, the years of experience, or the number of trainees, among the staff data of (4), is one example of track record information with regard to the assistance.

Further, information that contains index data including at least one of the symptom, the physical ability and the recovery level of the trainee 10, among the trainee data of (3), may be referred to as physical ability information of the trainee 10. In other words, the physical ability information may contain the symptom information, the Br. stage, the SIAS, the initial gait FIM, the latest gait FIM, and so on of the trainee 10. Note that the physical ability information may contain various data indicating the physical ability of the trainee 10. Further, such index data of the trainee 10 may be determined or calculated in the training device 20, or may be evaluated by a training staff such as a doctor or a physical therapist, input through an input device (not shown), and stored into a storage device in the training device 20. Further, the physical ability information may contain harness information about a harness used by the trainee. Note that the detection data is an example of the physical ability information, and it may be a set of measured values respectively corresponding to a plurality of indices (for example, a plurality of sensors) for measurement. Further, the detection data is data where a measured value is associated with measurement time and an index.

The latest gait FIM, etc. is generally contained in one of the physical ability information and the track record information; however, it may be contained in both of them because it can be included in both concepts of the physical ability information and the track record information.

The training device 20 may associate trainee attribute information such as the attributes, the physical information, and so on of the trainee 10 with the above-described physical ability information and store them into an internal storage device. The trainee attribute information may contain the age, the gender, the physical size (body height, weight, etc.), and so on of the trainee 10 among the trainee data of the above-described (3). Further, the trainee attribute information may contain the name or ID of the trainee 10, and the trainee attribute information may also contain preference information indicating the preference of the trainee 10, personality information indicating the personality of the trainee 10, and so on. Further, the FIM contained in the physical ability information or the track record information may contain exercise items different from those related to the ability to walk, and the FIM may also contain cognitive items. Note that a part or whole of the physical ability information or the trainee attribute information may be referred to as physical information, basic information, or trainee feature information and so on. The training device 20 may acquire the index data, the trainee attribute information and so on from an electronic health record system (not shown) of a medical institution, etc. or by input of a training staff.

Further, the training device 20 may associate the trainee attribute information, etc. with the physical ability information, such as detection data and index data, and store them into a storage device in the training device 20. The training device 20 may transmit the track record information, the physical ability information, and so on to the rehabilitation facility apparatus 30 through the network N, and store them into a storage device in the rehabilitation facility apparatus 30. Alternatively, the training device 20 may transmit the track record information, the physical ability information, and so on to the retrieval apparatus 50 through the network N in response to operation of a training staff, etc., detection of detection data, a predetermined interval, or an acquisition request from the retrieval apparatus 50.

The rehabilitation facility apparatus 30 is an information processing apparatus that is installed in the rehabilitation facility 61 where the trainee 10 undergoes rehabilitation by using the training device 20 with the assistance of the training assistant 11 or a facility that manages this rehabilitation, or, that is at least administered by this facility. The rehabilitation facility apparatus 30 is a database system that at least manages the track record information, the physical ability information, or the like.

For example, the rehabilitation facility apparatus 30 receives the physical ability information and the trainee attribute information acquired from the training device 20 through the network N, and stores the physical ability information and the trainee attribute information in association with each other into an internal storage device. Further, the rehabilitation facility apparatus 30 stores the track record information acquired from the training device 20 through the network N in association with each training assistant 11 into the internal storage device. Furthermore, the rehabilitation facility apparatus 30 may acquire part of the track record information and the physical ability information from the training device 20 through the network N and may acquire the rest of the information from an electronic health record system (not shown) of a medical institution, etc., or by input of the training staff. In the case where the trainee 10 performs training using a device different from the training device 20, the rehabilitation facility apparatus 30 may acquire data equivalent to the above-described data by input of the training staff.

Further, the rehabilitation facility apparatus 30 may transmit the track record information, the physical ability information, and so on to the retrieval apparatus 50 through the network N in response to operation of a training staff, etc., acquisition of the physical ability information, a predetermined interval, or an acquisition request from the retrieval apparatus 50. Note that the configuration of the rehabilitation facility apparatus 30 can be implemented by a known information system, etc., and the detailed description thereof is omitted.

The trainee terminal 40 is an information processing apparatus to be used by a user on the trainee side, including the trainee 10, a family member, a guardian, etc. of the trainee 10. The trainee terminal 40 has a communication function through the network N, and it may be a personal computer, or a portable information terminal such as a tablet terminal or a smartphone, for example. The trainee terminal 40 receives a search request to retrieve rehabilitation facilities through an input device in response to a user's operation on the trainee side, and transmits the search request to the retrieval apparatus 50 through the network N. Further, the trainee terminal 40 receives a search result for potential rehabilitation facilities from the retrieval apparatus 50 through the network N, and outputs (displays etc.) the result to an output device such as a screen.

Rehabilitation facilities 62 and 63 are examples of rehabilitation facilities different from the rehabilitation facility 61 where the trainee 10 has been undergoing rehabilitation. The number of rehabilitation facilities different from the rehabilitation facility 61 may be three or more. The rehabilitation facility 62, to which training assistants 6211 to 621x (x is a natural number of 1 or more) belong, includes one or more training device 622 and a rehabilitation facility apparatus 623. The training assistants 6211 etc. have the experience of assisting a plurality of trainees in rehabilitation, and the track record information and the physical ability information of the trainees are accumulated in the training device 622 or the rehabilitation facility apparatus 623. Then, the training device 622 or the rehabilitation facility apparatus 623 transmits the track record information, the physical ability information, and so on to the retrieval apparatus 50 through the network N, just like the training device 20 or the rehabilitation facility apparatus 30 described above. Likewise, the rehabilitation facility 63, to which training assistants 6311 to 631y (y is a natural number of 1 or more) belong, includes a training devices 632 and a rehabilitation facility apparatus 633. The rehabilitation facility 63 is the same as the rehabilitation facility 62, and the detailed description thereof is omitted.

The retrieval apparatus 50 is one or more information processing apparatus for a trainee who has undergone rehabilitation to search for a next rehabilitation facility that matches his/her needs for rehabilitation or physical ability. The retrieval apparatus 50 is a server device, for example, and it is assumed that a web server, an application server and a database server are running on an OS (Operating System), and a web application that implements object retrieval processing according to this embodiment is running on the application server. Note that, however, the software configuration of the retrieval apparatus 50 is not limited thereto.

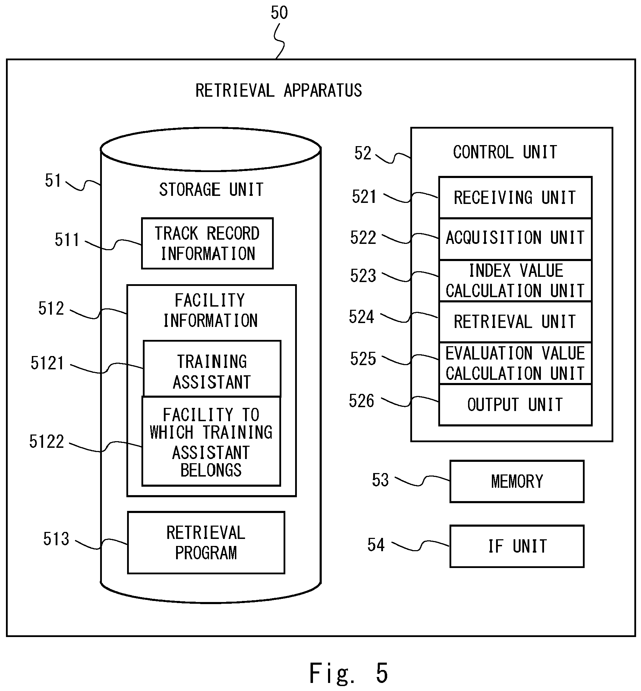

Fig. 5 is a block diagram showing the configuration of the retrieval apparatus 50 according to the first embodiment. Fig. 5 shows an internal configuration in the case where the retrieval apparatus 50 is configured using one computer device in the form of a functional block.

The retrieval apparatus 50 includes a storage unit 51, a control unit 52, a memory 53, and an IF unit 54. The storage unit 51 is a non-volatile storage device such as a hard disk or a flash memory, for example. The storage unit 51 at least stores track record information 511, facility information 512, and a retrieval program 513.

The track record information 511 is track record information of a plurality of training assistants with regard to the assistance of training, as described above. Particularly, the track record information 511 preferably contains the physical ability information before and after the recovery of trainees to whom the assistance has been provided in the past, and a recovery period. The track record information 511 may contain at least some of a measured value in the training device used for training, the physical ability information before and after training of a trainee, a detection value in the training device regarding the assistance by the training assistant, information about the details of rehabilitation, and evaluation of the training assistant by a trainee. The information about the details of rehabilitation may be identification information of a PT in charge of rehabilitation, a disease name, a symptom, etc. of a trainee, a body part rehabilitated, the object of rehabilitation (needs, such as being able to walk on his/her own, for example), the physical ability information before and after recovery (for example, a measured value (FIM etc.), a gait style before and after recovery etc.), a recovery period, recovery efficiency, and so on, for example, though not limited thereto. In the description of this embodiment, the track record information 511 is track record information regarding the assistance of training provided to a given trainee by each of the training assistants 6211 to 621x and the training assistants 6311 to 631y.

The facility information 512 is an example of the facility information for performing training, which indicates the facility to which each of a plurality of training assistants belongs. For example, the facility information 512 is information where a training assistant 5121 and a facility 5122 to which the training assistant belongs are associated with each other. The training assistant 5121 is at least identification information of the training assistant. The facility 5122 is identification information of the rehabilitation facility to which the training assistant 5121 belongs. When the training assistant 5121 belongs to a plurality of rehabilitation facilities, a plurality of facilities 5122 are associated with one training assistant 5121.

The retrieval program 513 is a computer program that performs processing for a method of retrieving rehabilitation facilities according to the first embodiment.

A memory 53 is a non-volatile storage device such as a RAM (Random Access Memory), and it is a storage area for temporarily storing information during operation of a control unit 52.

An IF unit 54 is an interface that inputs and outputs information to and from the outside of the retrieval apparatus 50. The IF unit 54 is a communication circuit for performing communication at least through the network N.

The control unit 52 is a processor that controls each component of the retrieval apparatus 50. The control unit 52 loads the retrieval program 513 from the storage unit 51 to the memory 53 and executes the retrieval program 513. The control unit 52 thereby implements the functions of a receiving unit 521, an acquisition unit 522, an index value calculation unit 523, a retrieval unit 524, an evaluation value calculation unit 525, and an output unit 526, which are described later.

The receiving unit 521 receives needs regarding training that is performed by the trainee 10 in order to restore or maintain the physical ability. The needs regarding training are information corresponding to the above-described object of rehabilitation, which is information indicating being able to walk on his/her own, for example. It is assumed that the receiving unit 521 typically receives a search request to retrieve rehabilitation facilities, and search condition in this search request include the needs regarding training. Further, the search request to retrieve rehabilitation facilities may contain the current physical ability information of the trainee 10.

The acquisition unit 522 acquires the track record information 511 from the outside. For example, as described above, the acquisition unit 522 collects the track record information related to each training assistant from the training device 20, the rehabilitation facility apparatus 30, the training device 622, the rehabilitation facility apparatus 623, the training device 632, the rehabilitation facility apparatus 633, and so on, through the network N, and stores the information as the track record information 511 into the storage unit 51. Alternatively, the acquisition unit 522 may receive input of the track record information by user operation through an input device directly connected to the retrieval apparatus 50 or a computer connected through a LAN etc.

The index value calculation unit 523 calculates, for each training assistant, an index value of the assisting ability related to the needs received by the receiving unit 521 from the track record information 511. Specifically, the index value calculation unit 523 determines a level of the assisting ability according to predetermined criteria of determination for each of the training assistants in the track record information 511. The predetermined criteria of determination may be to satisfy one or a plurality of the following conditions (a) to (d) in terms of the FIM efficiency, the walking speed, the walking stability, the gait style, and so on, for example. Note that, however, the criteria of determination are not limited thereto, and it may be, in the simplest example, the years of experience. The FIM efficiency is an example of a value indicating the recovery speed of a trainee.
(a) The average value or the maximum value of the FIM efficiently (for example, a period of time required until a trainee becomes able to walk without assistance, such as the length of a period required until the FIM reaches 6 points or more) of all trainees whom a target training assistant has assisted is equal to or less than a threshold.
(b) The average value or the minimum value of the walking speed of all trainees whom a target training assistant has assisted is equal to or more than a threshold. Or, the rate of increase of the walking speed is equal to or more than a threshold.
(c) The average value or the maximum value of the frequency of abnormal gait during level-ground walking (walking on the treadmill 131) of all trainees whom a target training assistant has assisted is equal to or less than a threshold. Or, the rate of decrease of the frequency is equal to or more than a threshold.
(d) An index of the elegance of gait (gait style) of all trainees whom a target training assistant has assisted is equal to or more than a threshold. Or, the rate of increase of this index is equal to or more than a threshold.

For each of the above-described (a) to (d), for the number m (m is a natural number of 2 or more) of levels, a threshold set made up of (m-1) number of thresholds is prepared. The threshold sets for the above-described (a) to (d) are different from one another. Although threshold processing is performed on the data of all trainees whom a target training assistant has assisted in the above-described (a) to (d), threshold processing may be performed on the data of all rehabilitation sessions which a target training assistant has assisted. The case where two or more training assistants assist one trainee in the same or different period of time is thereby taken into consideration.

Further, threshold processing may be executed on the rehabilitation data that distinguishes between rehabilitation which a training assistant has attended as a primary staff and rehabilitation which a training assistant has attended as an assistant training staff. Likewise, threshold processing may be performed on the rehabilitation data that distinguishes between rehabilitation which a training assistant has attended as a staff who uses the management monitor 139 and rehabilitation which a training assistant has attended as a staff who helps (holds) a trainee.

For example, the index value calculation unit 523 specifies, for each of the training assistants contained in the track record information 511, the track record information that corresponds to the needs received by the receiving unit 521, which is: (a) the FIM efficiently, (b) the average value etc. of the walking speed, (c) the average value etc. of the frequency of abnormal gait during level-ground walking, and (d) an index of the gait style. Then, for each specified value of the track record information, the index value calculation unit 523 determines whether a training assistant is skilled or not, assuming that the number of levels is 2 in all of the above-described (a) to (d), and then determines that a training assistant determined to be skilled in three or more conditions is at a level high enough to be determined as skilled. In this case, the index value calculation unit 523 can consider that the level determined for each training assistant is calculated as the index value. Alternatively, the index value calculation unit 523 may use only the above-described (a) as the condition and use "2" as the number of levels, and determine whether a training assistant is a skilled staff or not by determination using one threshold.

Alternatively, the index value calculation unit 523 may calculate the index value of each training assistant for each of the needs from the track record information 511 stored in the storage unit 51 on a regular basis, not limited to the timing when the receiving unit 521 receives a search request.

The index value of each training assistant is calculated for each of the needs because it is likely that a training assistant's specialty is different depending on disease or symptom. Symptom data indicating a disease or a symptom of the trainee 10 may be contained in the needs. The symptom data is data that describes the above-described symptom information. Particularly, in the case of gait training, symptoms to be contained in this symptom data may be backward movement of trunk, forward lean of trunk, affected side movement of trunk, flexion of knee joint, difficulty in toe-off, difficulty in swing through, backward lean of trunk, retraction of pelvis, forward lean of lower extremity, extension of knee joint, flexed position of knee joint, forward swing, and so on, for example. Further, symptoms to be contained in this symptom data may be non-affected side movement of trunk, equines gait, lift of pelvis, external rotation of hip joint, circumduction gait, medial whip, and so on, for example. The index value calculation unit 523 can thereby set, for each disease or symptom of the trainee 10, a higher level (index level) to a training assistant who is considered to be skilled in assisting the trainee with that disease or symptom.

Further, the index value calculation unit 523 may be configured to determine the above-described level for each value indicated by the index data, such as the initial FIM, of the trainee 10. Thus, for each value indicated by the index data, the index value calculation unit 523 can identify a training assistant who is considered to be skilled for a trainee with each value.

Furthermore, the index value calculation unit 523 may calculate, as the index value, the recovery efficiency of each training assistant based on the physical ability information and the recovery period before and after recovery.

The retrieval unit 524 searches for a plurality of potential training assistants who meet the needs based on the track record information 511. In this search, the retrieval unit 524 retrieves, among a plurality of training assistants, the training assistants whose index value is higher than a predetermined threshold as the potential training assistants. The retrieval unit 524 then identifies a facility to which each of the plurality of potential training assistants belongs by referring to the facility information 512.

The evaluation value calculation unit 525 performs predetermined statistical processing based on the track record information of each potential training assistant and thereby calculates an evaluation value for each facility. Particularly, it is preferred that the evaluation value calculation unit 525 calculates the evaluation value for each facility by performing statistical processing on the index value of each potential training assistant. The predetermined statistical processing may be an operation of calculating the average of the index value for each facility, the variance indicating variation of the index value, and so on, for example.

The output unit 526 outputs information based on the plurality of potential training assistants. The output unit 526 according to this embodiment outputs a list of facilities indicated by the identified facilities to which the training assistants belong. For example, the output unit 526 transmits the list of facilities to the trainee terminal 40 through the network N. Alternatively, the output unit 526 may display a search result on a display device, such as a screen, connected to the retrieval apparatus 50. Particularly, the output unit 526 outputs a list of facilities in accordance with an order of evaluation values. Specifically, the output unit 526 sorts the facilities indicated by the identified facilities to which the training assistants belong in descending order of the evaluation value, and outputs a result of sorting as the list of facilities. Further, the output unit 526 may output a list of facilities in which the facilities are divided by area. In this case, an area where the trainee 10 lives may be included in the search condition received by the receiving unit 521.

Note that each of the receiving unit 521, the acquisition unit 522, the index value calculation unit 523, the retrieval unit 524, the evaluation value calculation unit 525, and the output unit 526 described above may be implemented by dedicated hardware. Further, some or all of the elements of each device may be implemented by general-purpose or dedicated circuitry, processor, or a combination of them. They may be configured using a single chip, or a plurality of chips connected through a bus. Some or all of the elements of each device may be implemented by a combination of the above-described circuitry, etc. and a program. Further, a CPU (Central Processing Unit), a GPU (Graphics Processing Unit), an FPGA (field-programmable gate array), and so on may be used as a processor (the control unit 52).

In the case where some or all of the elements of the retrieval apparatus 50 are implemented by a plurality of information processing apparatuses, circuitries, and so on, the plurality of information processing apparatuses, circuitries, and so on may be centralized or dispersed. For example, the information processing apparatuses, circuitries and so on may be implemented as a form in which components are connected through a communication network, such as a client-server system or a cloud computing system. Further, the functions of the retrieval apparatus 50 may be provided in a SaaS (Software as a Service) format.

The storage unit 51 may be an external storage device of the retrieval apparatus 50, and it may input and output data to and from the retrieval apparatus 50 by a storage system, a database system, etc.

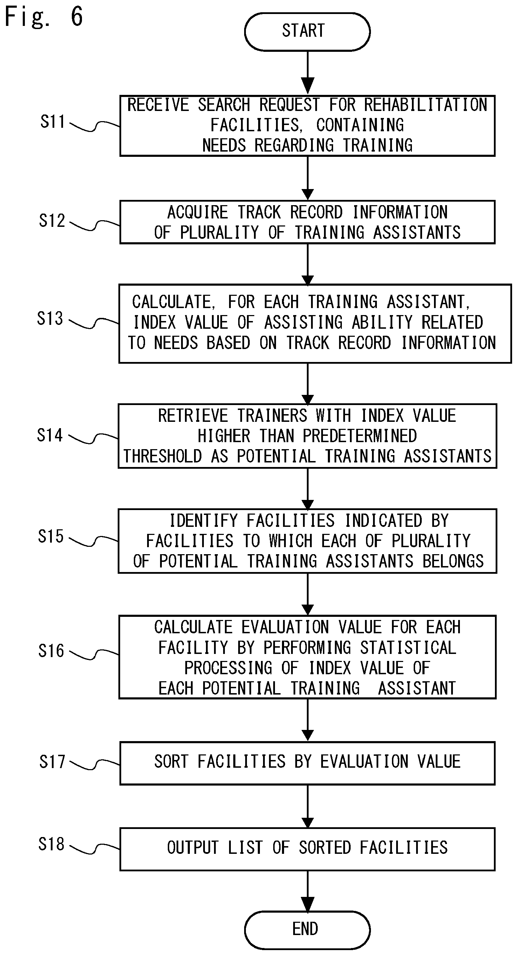

Fig. 6 is a flowchart showing the flow of a retrieval method according to the first embodiment. For example, a user on the trainee side enters a search request to retrieve rehabilitation facilities for the trainee 10 to effectively undergo rehabilitation by using the trainee terminal 40. It is assumed that the search request contains the needs regarding training as search condition. It may also contain the physical ability information, etc. of the trainee 10 as search condition. The trainee terminal 40 transmits the entered search request to the retrieval apparatus 50 through the network N. Alternatively, a user of the retrieval apparatus 50 enters the search request by using an information processing apparatus connected to the retrieval apparatus 50 in response to a request from a user on the trainee side, etc., and this information processing apparatus outputs the entered search request to the retrieval apparatus 50.

The receiving unit 521 receives the search request from an information terminal or an information processing apparatus such as the trainee terminal 40 or the rehabilitation facility apparatus 30 described above (S11).

Next, the acquisition unit 522 acquires the track record information 511 (S12). For example, the acquisition unit 522 reads the track record information 511 corresponding to the needs regarding training contained in the search request from the storage unit 51. Alternatively, the acquisition unit 522 transmits an acquisition request for the track record information that corresponds to the needs regarding training contained in the search request to the training device 622, the rehabilitation facility apparatus 623, the training device 632 and the rehabilitation facility apparatus 633 through the network N. The acquisition unit 522 then receives the track record information transmitted from the training device 622 etc. through the network N in response to the acquisition request.

Then, the index value calculation unit 523 calculates, for each training assistant, an index value of the assisting ability related to the needs based on the track record information (S13). For example, the index value calculation unit 523 identifies the track record information (for example, at least part of the information of the above-described (a) to (d)) corresponding to the needs for each of the training assistants contained in the track record information acquired in Step S12. The index value calculation unit 523 then determines whether the identified track record information exceeds a threshold or not, and calculates an index value of each training assistant based on a result of the determination.

Then, the retrieval unit 524 retrieves the training assistants whose index value is larger than a predetermined threshold as potential training assistants (S14). In this step, the retrieval unit 524 generally retrieves at least two potential training assistants.

The retrieval unit 524 then identifies facilities indicated by the facilities to which each of the plurality of potential training assistants belongs by referring to the facility information 512 (S15).

Then, the evaluation value calculation unit 525 performs statistical processing of the index value of each potential training assistant and thereby calculates an evaluation value for each facility (S16).

After that, the output unit 526 sorts the facilities based on the evaluation value (S17). For example, the output unit 526 sorts the facilities in descending order of the evaluation value. Then, the output unit 526 outputs a list of the sorted facilities (S18). For example, the output unit 526 transmits the list of the sorted facilities to the trainee terminal 40 that has made a search request through the network N. A user on the trainee side can thereby know the rehabilitation facilities to which the training assistants who suit the needs regarding training of the trainee 10 belong.

As described above, according to this embodiment, it is possible to retrieve a rehabilitation training assistant or a rehabilitation facility to which this training assistant belongs in order for a trainee to effectively undergo rehabilitation. This is because it is possible to identify a rehabilitation facility to which the training assistant who match the needs regarding training belongs from the track record information of rehabilitation of a plurality of training assistants and output them as a search result. Further, even when there are a plurality of training assistants who match the needs, by sorting them by the evaluation value for each facility and outputting the result, it is possible to show the priority level corresponding to the degree of match of each training assistant. Further, when searching for a training assistant who matches the needs, by using an index value indicating the level of each training assistant from the track record information corresponding to the needs, it is possible to identify a more appropriate training assistant.

### <Second Embodiment>

A second embodiment is a modified example of the above-described first embodiment. Note that the overall configuration of a retrieval system according to the second embodiment is the same as that of Fig. 4, and therefore the illustration of the same is omitted, and the description of the common elements is also omitted.

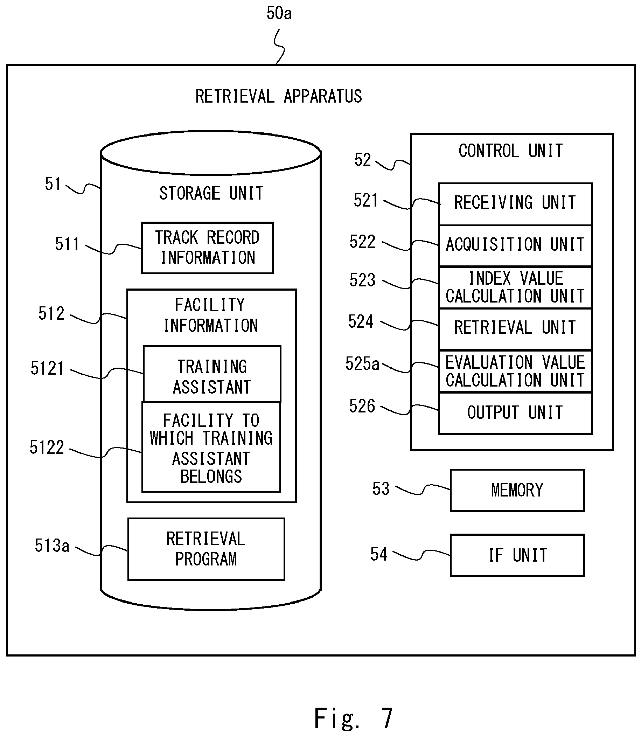

Fig. 7 is a block diagram showing the configuration of a retrieval apparatus 50a according to the second embodiment. Fig. 7 is different from Fig. 5 in that the retrieval program 513 and the evaluation value calculation unit 525 are replaced with a retrieval program 513a and an evaluation value calculation unit 525a. The other elements are the same as those of the first embodiment, and the description of the common elements is omitted.

The retrieval program 513a is a computer program that performs processing for a method of retrieving rehabilitation facilities according to the second embodiment. It is assumed that the track record information 511 at least contains, for each training assistant, the recovery efficiency of the physical ability of trainees to whom the assistance has been provided in the past. The evaluation value calculation unit 525a performs statistical processing on the recovery efficiency for each potential training assistant.

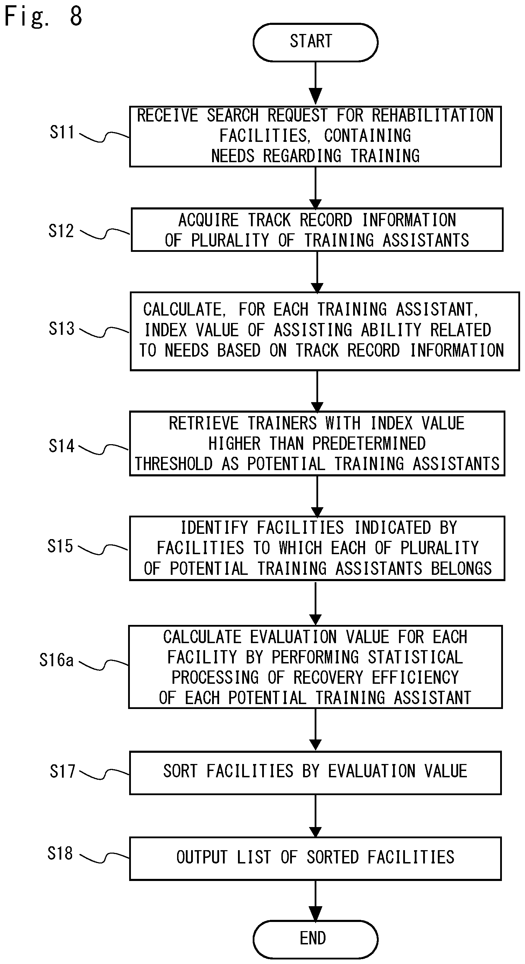

Fig. 8 is a flowchart showing the flow of a retrieval method according to the second embodiment. Fig. 8 is different from Fig. 6 in that Step S16 is replaced with S16a. The other steps are the same as those of Fig. 6, and the description thereof is omitted as appropriate.

After Step S15, the evaluation value calculation unit 525a performs statistical processing on the recovery efficiency of each potential training assistant and thereby calculates the evaluation value for each facility (S16a). It is thereby possible to evaluate the facilities by the more direct and typical assisting ability that suites the needs regarding training.

### <Third Embodiment>

A third embodiment is a modified example of the above-described first or second embodiment. The overall configuration of a retrieval system according to the third embodiment is the same as that of Fig. 4, and it is not shown in the drawings, and the description of the common elements is omitted.

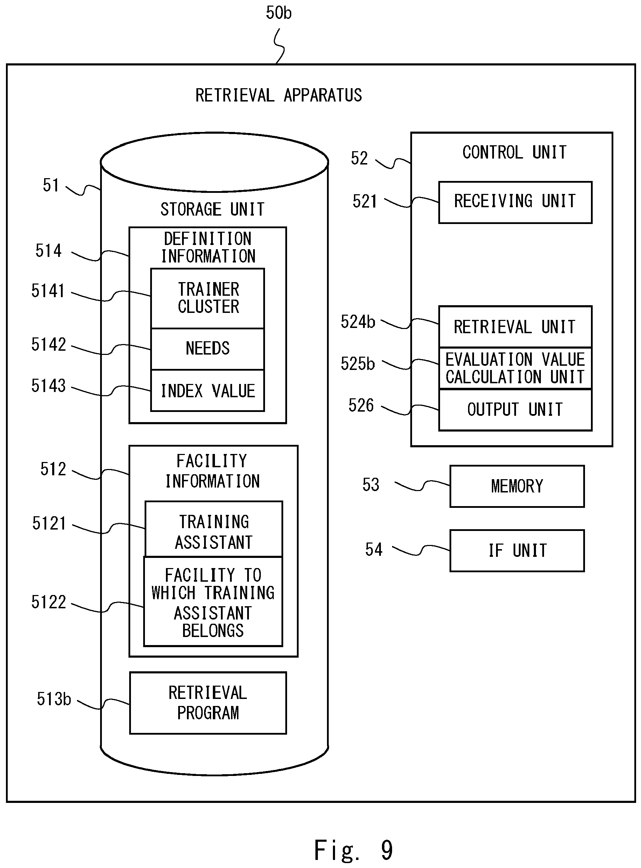

Fig. 9 is a block diagram showing the configuration of a retrieval apparatus 50b according to the third embodiment. Fig. 9 is different from Fig. 5 in that the track record information 511, the retrieval program 513, the retrieval unit 524 and the evaluation value calculation unit 525 are replaced with definition information 514, a retrieval program 513b, a retrieval unit 524b and an evaluation value calculation unit 525b, and the acquisition unit 522 and the index value calculation unit 523 are eliminated. The other elements are the same as those of the first embodiment, and the description of the common elements is omitted.

The retrieval program 513b is a computer program that performs processing for a method of retrieving rehabilitation facilities according to the third embodiment. The storage unit 51 may store the track record information 511.

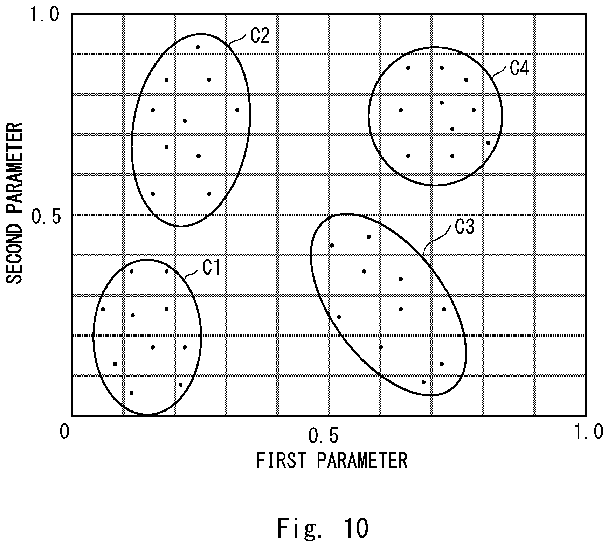

The definition information 514 is information that associates clusters of training assistants classified by cluster analysis of the track record information with the demands. Fig. 10 is a view showing an example of clusters of training assistants classified by cluster analysis of the track record information. For example, it is assumed that the control unit 52 of the retrieval apparatus 50b includes an analysis unit that performs cluster analysis. It is also assumed that the track record information according to this embodiment contains the above-described rehabilitation data, which is, at least some of (1) configuration parameters of the gait training device 100, (2) detection data detected by a sensor, etc. in the gait training device 100, (3) data related to the trainee 900, and (4) data related to the training staff 901. Particularly, the rehabilitation data at least contains the staff data indicating the training staff 901 who has assisted the trainee 900 in rehabilitation undergone by the trainee 900 by using the gait training device 100. Further, the rehabilitation data at least contains behavior data indicating the assisting behavior performed by the training staff 901 for the purpose of assisting the trainee 900 and index data indicating the recovery level of the trainee 900.

The analysis unit performs cluster analysis of the rehabilitation data collected in the same manner as in the first or second embodiment, and thereby classifies the training staffs. The cluster analysis in the analysis unit may use k-means, for example. Each of clusters as an analysis result indicates the classified tendency of the rehabilitation data, and it is preferably adjusted so that each group of data classified by the skill level of the training staff corresponds thereto.

The cluster analysis in the analysis unit may also use X-means, which extends the k-means and is capable of automatically setting the number of clusters. Further, the cluster analysis in the analysis unit may use various other techniques, such as GMM (Gaussian Mixture Models) capable of obtaining the probability density distribution, and spectral clustering that conducts clustering by focusing on the connectivity. In the spectral clustering, data is first converted into a graph, and ε-nearest neighbor, k-nearest neighbor (k-NN), total binding, and so on, may be used for this conversion.

To simplify the description, Fig. 10 shows an example of a result of performing cluster analysis on two parameters (two items) in the rehabilitation data. In the example of Fig. 10, as a result of cluster analysis of the rehabilitation data where the number of clusters (groups of data) is set to 4, the rehabilitation data is classified into clusters C1 to C4. Because the number of parameters (the number of spatial axes) in the cluster analysis typically corresponds to the number of items of the rehabilitation data, it is three or more in this embodiment.

Then, the definition information 514 according to this embodiment is information that associates training assistant clusters 5141, which are clusters C1 to C4, needs 5142 suitable for each of the clusters, and index values 5143 that represent training assistants who belong to the respective clusters. The needs 5142 suitable for the training assistant clusters 5141 may be determined by a user. Further, the index values 5143 may be calculated as index values related to the needs 5142 for each of the training assistants by the above-described index value calculation unit 523, and obtained as the average of the index value of the training assistants who belong to each cluster. Note that, however, a set of the needs 5142 and the index values 5143 associated with the training assistant cluster 5141 may be defined arbitrarily.

The retrieval unit 524b retrieves the training assistant cluster 5141 associated with the needs as a plurality of potential training assistants from the definition information 514. The retrieval unit 524b then identifies the facilities to which each of the plurality of potential training assistants belongs by referring to the facility information 512.

The evaluation value calculation unit 525b performs predetermined statistical processing of the index value 5143 of each of the potential training assistants and thereby calculates an evaluation value for each facility.

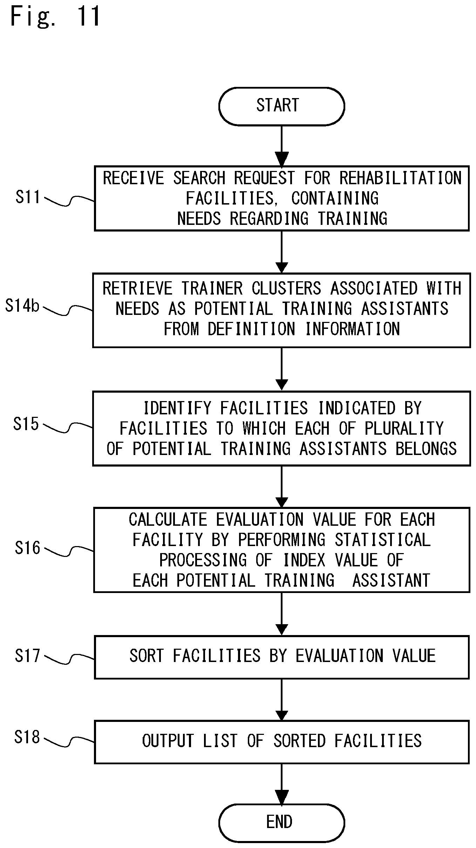

Fig. 11 is a flowchart showing the flow of a retrieval method according to the third embodiment. Fig. 11 is different from Fig. 6 in that Steps S12 and S13 are omitted, and Step S14 is replaced with S14b. The other steps are the same as those of Fig. 6, and the description thereof is omitted as appropriate.

After Step S11, the retrieval unit 524b retrieves the training assistant cluster 5141 associated with the needs received in Step S11 as a plurality of potential training assistants from the definition information 514 (S14b). Specifically, the retrieval unit 524b sets a set of the training assistants who belong to the retrieved training assistant clusters 5141 as a plurality of potential training assistants. The retrieval unit 524b then identifies facilities indicated by the facilities to which each of the plurality of potential training assistants retrieved in Step S14b belongs by referring to the facility information 512 (S15).

As described above, according to the third embodiment, it is possible to retrieve the training assistant cluster that meets the needs by using the predetermined definition information 514, and calculate the evaluation value of each facility by using the index value in the training assistant cluster. It is thereby possible to increase the speed and efficiency of processing of identifying potential training assistants who suite the needs at the time of retrieval.

### <Other Embodiments>

Although the output unit outputs a list of facilities based on a plurality of potential training assistants in the above-described embodiments, the present disclosure is not limited thereto. For example, the output unit may output a plurality of potential training assistants. This also allows a user on the trainee side to find the training assistants who meet the needs regarding training and identify a rehabilitation facility based on the training assistants. Further, the output unit may output, in addition to a list of facilities, a list of training assistants who belong to those facilities and more effectively meet the needs. A user on the trainee side can thereby select a facility in a comprehensive manner, including the number of training assistants who belong to the facility and the index value of each training assistant.

Particularly, the output unit may assign levels to a plurality of potential training assistants according to the index values, and output the assigned level to each of the potential training assistants. The PT can thereby know at which level his/her track record of rehabilitation is ranked among PTs regarding the needs of rehabilitation. This contributes to the self-development of PTs in the rehabilitation capability (assisting ability). In this case, the privacy of a PT is protected by disclosing the ranked level only to this PT.

Further, the receiving unit according to the above-described embodiments may further receive the current physical ability information of a trainee, and the retrieval unit may retrieve a plurality of potential training assistants who meet the needs by further taking the received physical ability information into consideration. It is thus possible to identify more appropriate training assistants according to the degree of recovery of the current physical ability of the trainee and thereby retrieve more appropriate rehabilitation facilities.

Furthermore, when the evaluation value calculation unit according to the above-described embodiments calculates the evaluation value of each facility, it may take the track record information or the index value of other training assistants who belong to this facility and who are different from the potential training assistants. At this time, the statistical processing preferably assigns a higher weight to the index value, etc. of the potential training assistants than the other training assistants different from the potential training assistants. The proportion of the potential training assistants to the training assistants who belong to the facility is thereby also taken into consideration, and it is thereby possible to retrieve more appropriate rehabilitation facilities.

It should be noted that the present disclosure is not limited to the above-described embodiments and may be varied in many ways within the scope of the present disclosure. For example, although the present disclosure is described as a hardware configuration in the above embodiments, the present disclosure is not limited thereto. The present disclosure may be implemented by causing a CPU (Central Processing Unit) to execute a computer program to perform arbitrary processing.

In the above example, the program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g. magneto-optical disks), CD-ROM (compact disc read only memory), CD-R (compact disc recordable), CD-R/W (compact disc rewritable), and semiconductor memories (such as mask ROM, PROM (programmable ROM), EPROM (erasable PROM), flash ROM, RAM (random access memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g. electric wires, and optical fibers) or a wireless communication line.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. A retrieval system (1000) comprising:
a receiving unit (521) configured to receive needs regarding training to be performed by a trainee to restore or maintain his/her physical ability;
a retrieval unit (524) configured to retrieve a plurality of potential training assistants who meet the needs based on track record information of a plurality of training assistants with regard to assistance in the training; and
an output unit (526) configured to output information based on the plurality of potential training assistants.

2. The retrieval system (1000) according to Claim 1, further comprising:
a storage unit (51) configured to store facility information for performing the training, indicating a facility to which each of the plurality of training assistants belongs, wherein
the retrieval unit (524) identifies a facility to which each of the plurality of potential training assistants belongs by referring to the facility information, and
the output unit (526) outputs a list of facilities indicated by the identified facility.

3. The retrieval system (1000) according to Claim 2, further comprising:
an evaluation value calculation unit (525) configured to calculate an evaluation value for each facility by performing predetermined statistical processing based on the track record information of each of the potential training assistants,
wherein the output unit (526) outputs the list of the facilities in accordance with an order of the evaluation values.

4. The retrieval system (1000) according to Claim 3, further comprising:
an index value calculation unit (523) configured to calculate, for each of the training assistants, an index value of assisting ability related to the needs from the track record information,
wherein the evaluation value calculation unit (525) calculates the evaluation value for each facility by performing the statistical processing of the index value of each of the potential training assistants.

5. The retrieval system (1000) according to Claim 4, wherein the retrieval unit (524) retrieves training assistants with the index value higher than a predetermined threshold as the potential training assistants from the plurality of training assistants.

6. The retrieval system (1000) according to Claim 4 or 5, wherein
the track record information contains physical ability information before and after recovery of trainees to whom the assistance has been provided in the past and a recovery period, and
the index value calculation unit (523) calculates, as the index value, recovery efficiency of each of the training assistants based on the physical ability information before and after recovery and the recovery period.

7. The retrieval system (1000) according to any one of Claims 3 to 5, wherein
the track record information contains recovery efficiency of physical ability of trainees to whom the assistance has been provided in the past, and
the index value calculation unit (523) performs the statistical processing of the recovery efficiency of each of the potential training assistants.

8. The retrieval system (1000) according to any one of Claims 2 to 7, wherein the output unit (526) outputs the list of facilities where the facilities are divided by area.

9. The retrieval system (1000) according to any one of Claims 1 to 3, comprising:
an index value calculation unit (523) configured to calculate, for each of the training assistants, an index value of assisting ability related to the needs from the track record information,
wherein the retrieval unit (524) retrieves training assistants with the index value higher than a predetermined threshold as the potential training assistants from the plurality of training assistants.

10. The retrieval system (1000) according to Claim 9, wherein the output unit (526) assigns levels to the plurality of potential training assistants according to the index values, and outputs an assigned level to each of the potential training assistants.

11. The retrieval system (1000) according to any one of Claims 1 to 10, further comprising:
an acquisition unit (522) configured to acquire the track record information from an outside.

12. The retrieval system (1000) according to Claim 1, further comprising:
a storage unit (51) configured to store definition information associating a cluster of the training assistants classified by cluster analysis of the track record information with the needs,
wherein the retrieval unit (524) retrieves, from the definition information, a cluster of the training assistants associated with the needs as the plurality of potential training assistants.

13. The retrieval system (1000) according to Claim 12, wherein
the storage unit (51) further stores facility information for performing the training, indicating a facility to which each of the plurality of training assistants belongs, and an index value of assisting ability related to the needs, of each of the training assistants classified into a cluster of the training assistants,
the retrieval unit (524) identifies a facility to which each of the plurality of potential training assistants belongs by referring to the facility information,
the retrieval system (1000) further comprises an evaluation value calculation unit (525) configured to calculate an evaluation value for each facility by performing predetermined statistical processing of the index value of each of the potential training assistants, and
the output unit (526) outputs a list of facilities indicated by the identified facility in order of the evaluation value.

14. A retrieval method, in a computer, comprising:
receiving needs regarding training to be performed by a trainee to restore or maintain his/her physical ability;
retrieving a plurality of potential training assistants who meet the needs based on track record information of a plurality of training assistants with regard to assistance in the training; and
outputting information based on the plurality of potential training assistants.

15. A retrieval program causing a computer to execute:
receiving processing of receiving needs regarding training to be performed by a trainee to restore or maintain his/her physical ability;
retrieval processing of retrieving a plurality of potential training assistants who meet the needs based on track record information of a plurality of training assistants with regard to assistance in the training; and
output processing of outputting information based on the plurality of potential training assistants.
